# EUROPEAN PATENT APPLICATION

(11) **EP 3 792 358 A1**
(43) Date of publication of application: **17.03.2021**
(21) Application number: 19800559.7
(22) Date of filing: 10.05.2019
(51) Int. Cl.: C12N 15/09, C07K 16/00, C07K 19/00, C12P 21/00, C12N 15/62

(54) **ARTIFICIAL PROTEIN CONTAINING ANTIGEN-BINDING REGION OF ANTIBODY AND BEING FUSED WITH PHYSIOLOGICALLY ACTIVE PEPTIDE**

(30) Priority: 10.05.2018 US 201862669468 P
(71) Applicant: Mirabiologics Inc., Tokyo 153-0041 (JP)
(72) Inventor: TAKAGI, Junichi, Tokyo 153-0041 (JP); HIRAI, Hidenori, Tokyo 153-0041 (JP)
(74) Representative: D Young & Co LLP
(86) International application number: PCT/JP2019/018839
(87) International publication number: WO 2019/216436

(57) **Abstract**

The purpose of the present invention is to provide an artificial protein having a bioactive peptide fused with a site, of a protein having at least an antigen binding region of an antibody, present in a folded portion of a secondary structure in a structural region having an antigen-binding activity and exposed on a surface of the protein.

## Description

### Technical Field

The present invention relates to an artificial protein containing an antigen-binding region of an antibody and fused with a bioactive peptide, and the like.

### Background Art

An antibody has essentially a function of specifically binding to only one antigen molecule as a target molecule. There is known an antibody that recognizes two or more respectively different antigen molecules (multispecific antibody: MsAb) as a modified type antibody obtained by a protein engineering method.

A MsAb is an antibody simultaneously binding to two or more target molecules so that it can be used as a medicament that crosslinks cells of different kinds, for example, cancer cells and lymphocytes and thereby exhibiting an anti-cancer activity. In addition, a MsAb obtained by imparting an antibody having a first binding specificity inherent in an antibody with a second binding specificity can be accumulated on desired tissues or cells. In contrast to a medicament using a conventional monoclonal antibody having binding ability and/or inhibiting ability only against a single target, a MsAb is capable of controlling a disease via a complex action mechanism and is therefore regarded as important as a next-generation leading antibody medicament.

As the form of a MsAb, a bispecific antibody (BsAb) obtained by using, in combination, two antigen recognition Fab regions derived from respectively different antibodies is mainstream but in an IgG type BsAb, special engineering modification is necessary for heterodimerization of heavy chains derived from respectively different antibodies (Non-Patent Document 1). A technology called Fcab generates an IgG type BsAb by embedding second binding specificity in Fc, a region other than a Fab region, in order to evade problems in heterodimerization (Non-Patent Document 2). In Fcab, since a Fab region is still that of the original antibody, the first binding specificity which the antibody originally has is retained and the second binding specificity incorporated in Fc is simply added thereto.

However, the IgG type BsAb requires high-level engineering modification and has many difficulties in development or production, because it is an IgG type high molecule (molecular weight: 150000). Further, only one binding specificity is usually added to such IgG type BsAb. There is therefore no example of achieving addition of third and fourth binding specificities.

In contrast to these IgG type BsAbs, a tandem scFv (taFv) having Fv regions of two antibodies linked in series to each other as a single-chain type scFv is developed as a BsAb which is more compact and can be produced easily by gene recombination. It is used as a cancer immunotherapeutic agent (Bispecific T-cell Engager; BITE) that allows lymphocytes to accumulate on cancer cells and attack them (for example, refer to Patent Document 1). The taFv is however an artificial product having a structure far different from that of a naturally occurring antibody so that it has low stability in vivo (Non-Patent Document 3). Further, although three or more scFvs can be linked to one another in principal, the resulting antibody is expected to have more deteriorated stability so that the number of specificity added is limited to two at present.

On the other hand, Fab which is a portion of the original antibody molecule is composed of four domains, that is, VH, VL, CH1, and CL and has been used for long years as a stable antigen-binding protein available by treating IgG with a protease such as papain. The antigen-binding site of Fab (complementarity determining region: called "CDR") has three loop regions at each tip portion of VH and VL, six loops in total, so that there is room for engineering modification of regions other than these tip portions. This means that if a novel bioactive site is created by modifying the amino acid sequence of a region other than CDR, a novel function such as second target binding property can be given to an existing antibody. However, even if the amino acid of these loop regions is randomized to give a novel bioactivity, it usually sacrifices the structural stability largely so that it has more difficulty in creation than the above-described Fcab or taFv. As a conventional example of giving a new activity to a Fab region, only a light chain having a bioactive peptide or a small protein fused with the C terminal thereof is known (Patent Document 2).

### Citation List

### Patent Documents

Patent Document 1: Published Japanese translation of a PCT patent application No. 2018-525347
Patent Document 2: Published Japanese translation of a PCT patent application No. 2011-509084

### Non-Patent Documents

Non-Patent Document 1: Nature Biotechnology, 1998; 16: 677-681
Non-Patent Document 2: Protein Eng. Des. Sel., 2010; 23(4): 289-297
Non-Patent Document 3: Clin. Pharmacokinet., 2016; 55: 1271-1288

### Summary

### Technical Problem

An object of the present invention is to provide a novel artificial protein containing an antigen-binding region of an antibody and fused with a bioactive peptide.

### Solution to Problem

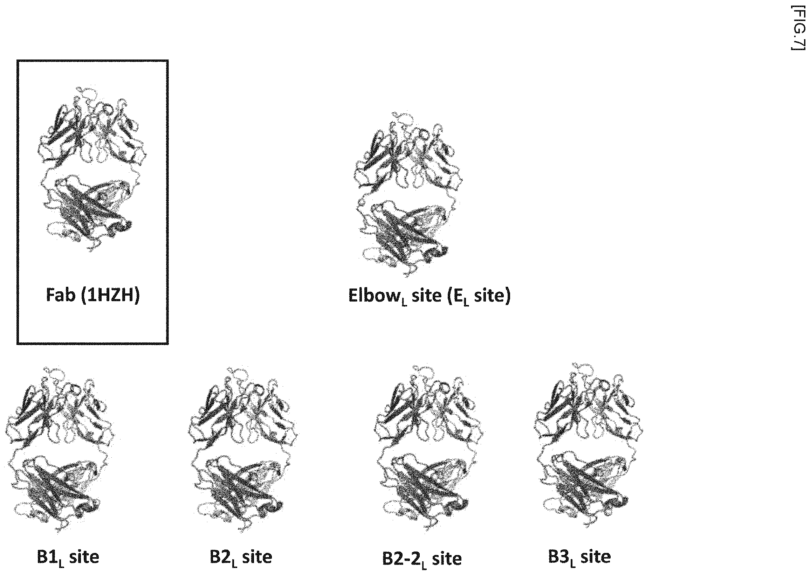

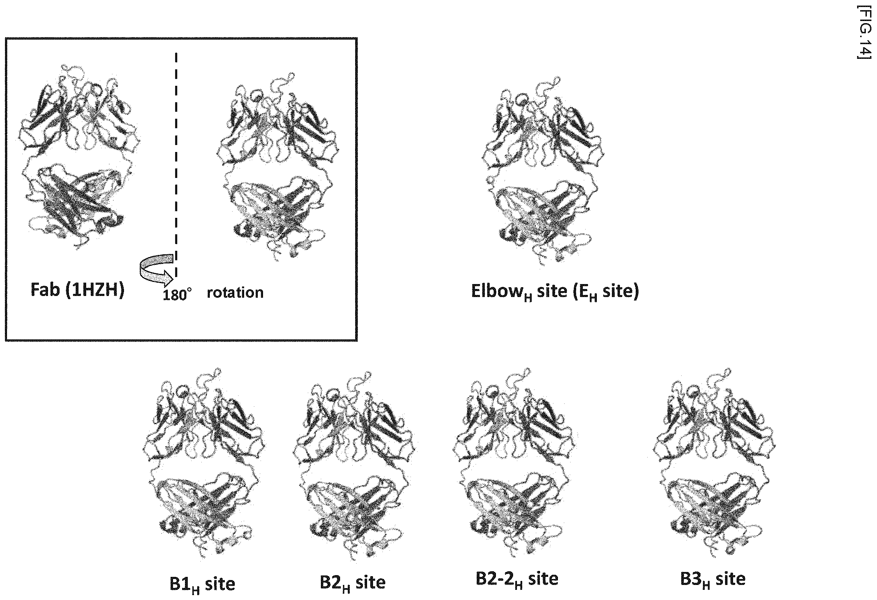

The present invention is as follows.
(1) An artificial protein having a bioactive peptide fused with a site, of a protein having at least an antigen-binding region of an antibody, present in a folded portion of a secondary structure in a structural region having an antigen-binding activity and exposed on a surface of the protein.
(2) The artificial protein as described in (1), retaining the antigen-binding activity derived from the antibody and endowed with a function derived from the bioactive peptide.
(3) The artificial protein as described in (1) or (2), wherein the bioactive peptide is a cyclic peptide or a linear peptide.
(4) The artificial protein as described in any of (1) to (3), wherein the protein having at least an antigen-binding region of an antibody is Fv, Fab, scFv, IgG, Diabody, or taFv.
(5) The artificial protein as described in any of (1) to (4), wherein the site present in a folded portion of a secondary structure in a structural region having an antigen-binding activity and exposed on a surface of a protein is present in a loop region or an elbow region in an antibody Fab fragment (Fab).
   As the loop region in an antibody Fab fragment is preferably an AB loop (CH1), CD loop (CH1), EF loop (CH1), AB loop (VH), C"D loop (VH), EF loop (VH), DE loop (CH1), FG loop (CH1) or CC' loop (VH), AB loop (CL), CD loop (CL), EF loop (CL), AB loop (VL), C"D loop (VL), EF loop (VL), DE loop (CL), FG loop (CL), or CC' loop (VL), while the elbow region is preferably a GA loop (VH-CH1) or GA loop (VL-CL).
   In the antibody Fab fragment, according to the Chothia number, the loop region of the H chain is preferably that present at positions 112-119, positions 125-134, positions 155-162, positions 186-194, positions 13-17, positions 61-66, positions 82b- 87, positions 172-174, positions 201-203, or positions 40-44 and the loop region of the L chain is preferably that present at positions 105 -113, positions 119-128, positions 151-158, positions 182-190, positions 13-18, positions 57-61, positions 76-83, positions 165-174, positions 198-203, or positions 39-44.
(5-1) The artificial protein described in (5), wherein an insertion site in the loop region of the antibody is a B1, B2, B2-2, B3, M1, M2, M2-2, M3, M3-2, M4, M5, M5-2, or M6 site in the light chain (L chain) shown in Fig. 7 and Fig. 8; or a B1, B2, B2_2, B3, M1, M2, M2-2, M3, M3-2, M4, M5, or M6 site in the heavy chain (H chain) shown in Fig. 14 and Fig. 15.
(5-2) The artificial protein as described in (5), wherein the insertion site in the elbow region of the antibody is an elbow site in the L chain shown in Fig. 7 or an elbow site in the H chain shown in Fig. 14.
(6) The artificial protein as described in any of (1) to (5-2), having multispecificity.
(7) A method of presenting a bioactive peptide on a protein having at least an antigen-binding region of an antibody, including fusing the bioactive peptide with a site of the protein present in a folded portion of a secondary structure in a structural region having an antigen-binding activity and exposed on a surface of the protein.
(8) The method of presenting a bioactive peptide on a protein as described in (7), including allowing the protein to retain an antigen-binding activity derived from the antibody and giving a function derived from the bioactive peptide to the protein.
(9) The method of presenting a bioactive peptide on a protein as described in (7) or (8), wherein the bioactive peptide is a cyclic peptide or a linear peptide.
(10) The method of presenting a bioactive peptide on a protein as described in any of (7) to (9), wherein the protein having at least an antigen-binding region of an antibody is Fv, Fab, scFv, IgG, Diabody, or taFv.
(11) The method of presenting a bioactive peptide on a protein as described in any of (7) to (10), wherein the site present in a folded portion of a secondary structure in a structural region having an antigen-binding activity and exposed on a surface of the protein is present in a loop region or an elbow region in an antibody Fab fragment (Fab).

As the loop region in the antibody Fab fragment is preferably an AB loop (CH1), CD loop (CH1), EF loop (CH1), AB loop (VH), C"D loop (VH), EF loop (VH), DE loop (CH1), FG loop (CH1) or CC' loop (VH), AB loop (CL), CD loop (CL), EF loop (CL), AB loop (VL), C"D loop (VL), EF loop (VL), DE loop (CL), FG loop (CL), or CC' loop (VL), while the elbow region is preferably a GA loop (VH-CH1) or GA loop (VL-CL).

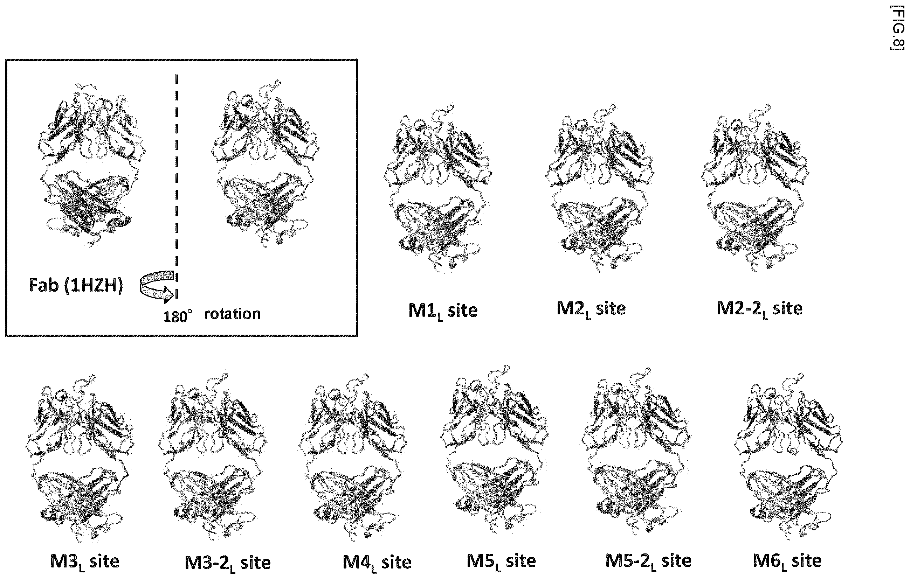

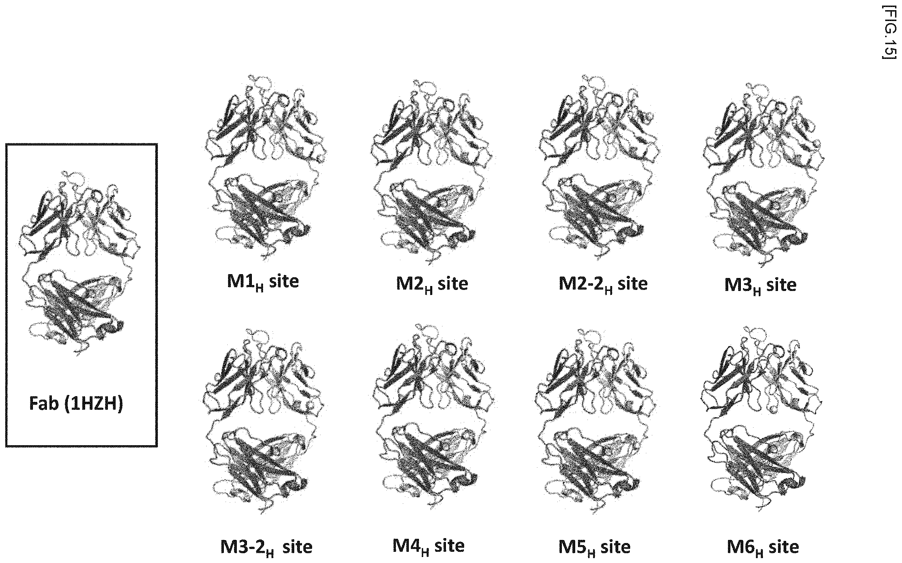

In the antibody Fab fragment, according to the Chothia number, the loop region of the H chain is preferably that present at positions 112-119, positions 125-134, positions 155-162, positions 186-194, positions 13-17, positions 61-66, positions 82b- 87, positions 172-174, positions 201-203, or positions 40-44 and the loop region of the L chain is preferably that present at positions 105 -113, positions 119-128, positions 151-158, positions 182-190, positions 13-18, positions 57-61, positions 76-83, positions 165-174, positions 198-203, or positions 39-44.
(11-1) The method of presenting a bioactive peptide on a protein as described in (11), wherein an insertion site in the loop region of the antibody is a B1, B2, B2-2, B3, M1, M2, M2-2, M3, M3-2, M4, M5, M5-2, or M6 site in the L chain shown in Fig. 7 and Fig. 8; or a B1, B2, B2-2, B3, M1, M2, M2-2, M3, M3-2, M4, M5, or M6 site in the H chain shown in Fig. 14 and Fig. 15.
(11-2) The method of presenting a bioactive peptide on a protein as described in (11), wherein the insertion site in the elbow region of the antibody is an elbow site in the L chain shown in Fig. 7 or an elbow site in the H chain shown in Fig. 14.

### Advantageous Effects of Invention

According to the present invention, a novel artificial protein including an antigen-binding region of an antibody and fused with a bioactive peptide can be provided.

### Brief Description of Drawings

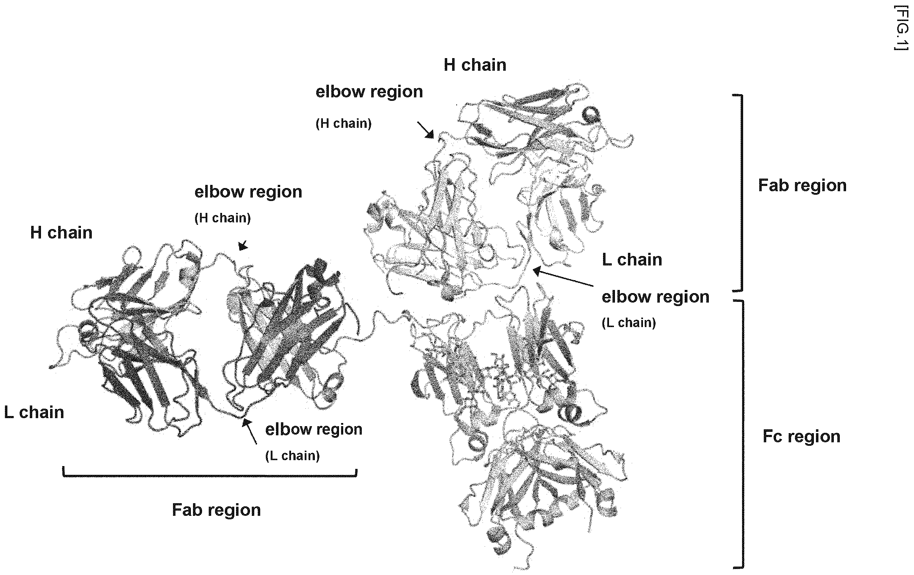

Fig. 1 shows a steric structure of 1HZH which is a human IgG antibody. A region connecting a VH domain and a CH1 domain and a region connecting a VL domain and a CL domain, in a Fab region, of an H chain and an L chain of an antibody, respectively, is shown as an elbow region.

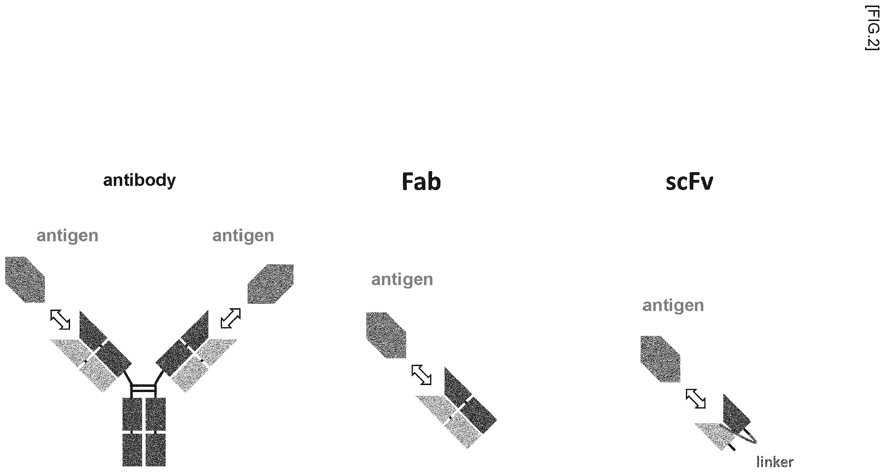

Fig. 2 schematically shows the respective structures of an antibody, Fab, and scFv as an example of a protein having at least an antigen-binding region of an antibody. Structures of Fab and scFv derived from the heavy chain and the light chain are shown while making them correspondent to the structure of the heavy chain and the light chain of the antibody. Among them, the scFv is a single-chain variable region fragment in which a variable region (Fv) composed of the VH domain derived from a heavy chain and the VL domain derived from a light chain are joined via a flexible peptide linker (shown as a linker). In addition, this drawing schematically shows binding manners of an antibody, Fab, and scFv to an antigen.

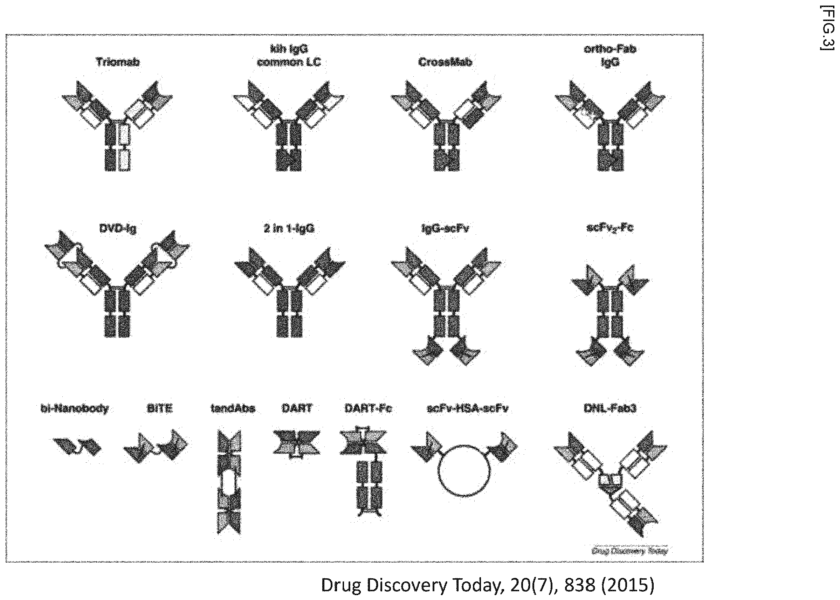

Fig. 3 schematically shows the structures of BsAb as an example of the protein having at least an antigen-binding region of an antibody. This BsAb has a structure making use of an existing antibody or antibody fragment and the structure can have many different designs.

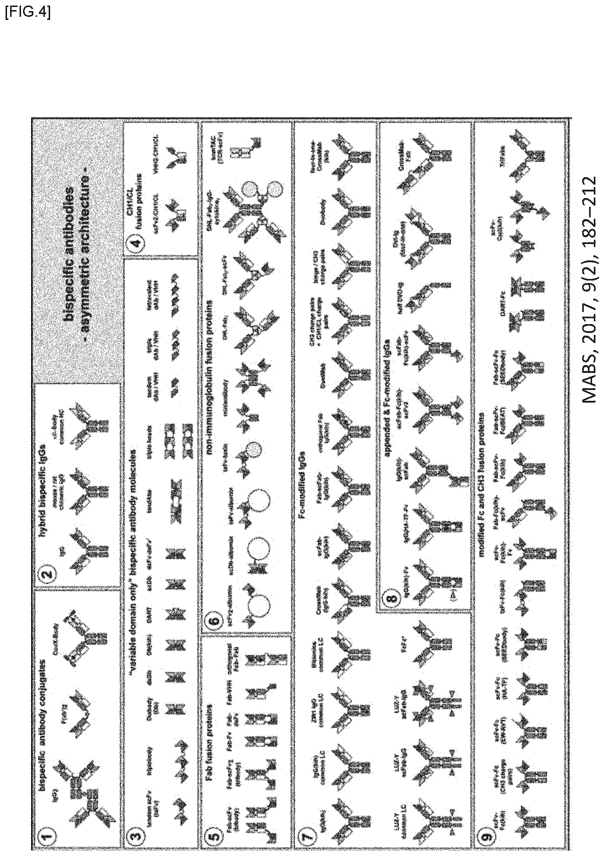

Fig. 4 schematically shows asymmetric BsAb structures as an example of the protein having at least an antigen-binding region of an antibody.

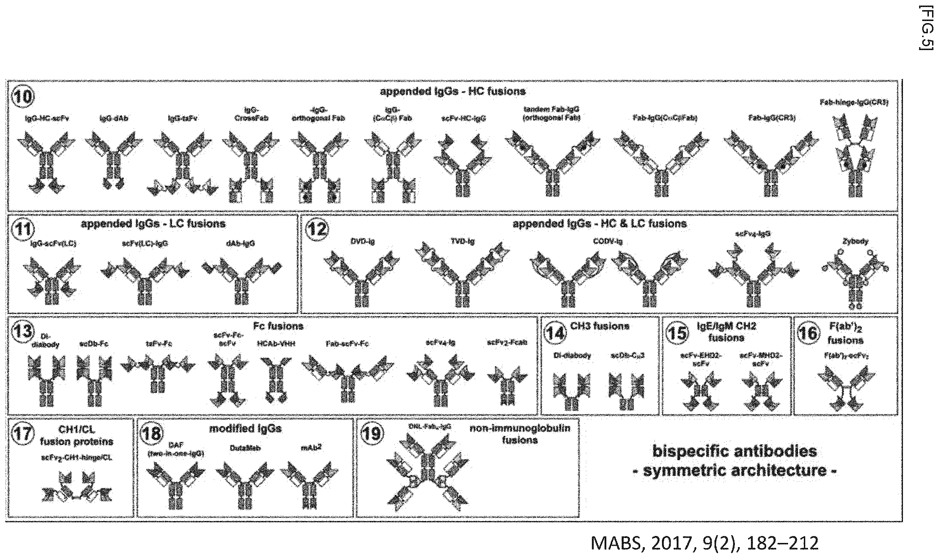

Fig. 5 schematically shows symmetric BsAb structures as an example of the protein having at least an antigen-binding region of an antibody.

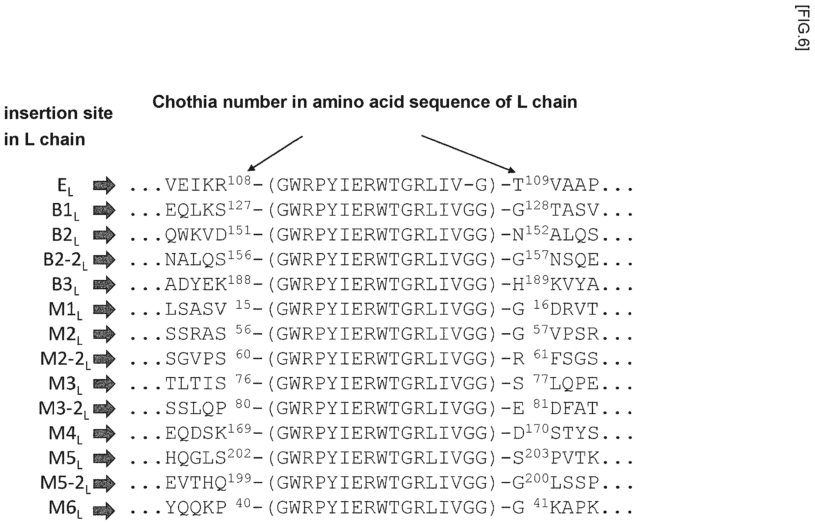

Fig. 6 shows an insertion site of a bioactive peptide in an L chain. The number of an amino acid residue is according to numbering based on the Chothia amino acid residue number. The inserted bioactive peptide corresponds to the amino acid sequence of mP6-9.
Fig. 7 shows a steric structure of the Fab region of 1HZH, a human IgG antibody. The structure placed on the nearer side shows an L chain and that placed on the far side shows an H chain. As insertion sites of a bioactive peptide in the L chain, shown are an elbow (E) site, B1 site, B2 site, B2-2 site, and B3 site.
Fig. 8 shows a steric structure of the Fab region of 1HZH, a human IgG antibody. It is a drawing obtained by rotating the steric structure of the Fab region shown in Fig. 7 by 180°. The structure placed on the nearer side shows an H chain and the structure placed on the far side shows an L chain. As insertion sites of a bioactive peptide in the L chain, shown are an M1 site, M2 site, M2-2 site, M3 site, M3-2 site, M4 site, M5 site, M5-2 site, and M6 site.

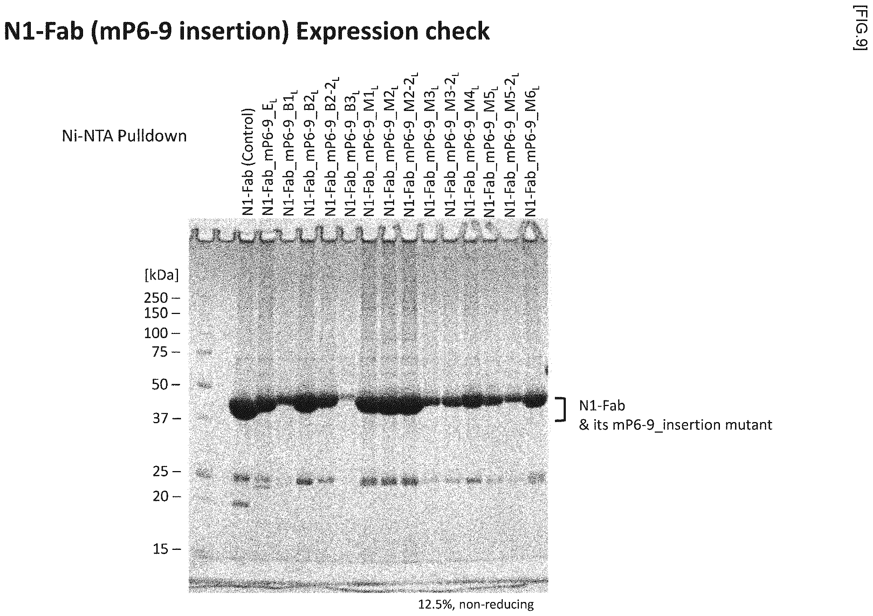

Fig. 9 shows the results of an Ni-NTA Pulldown test on the expression of the Fab (mP6-9 peptide fusion type N1_Fab) of a human IgG1 monoclonal antibody (N1 antibody, clone name: YW64.3) against neuropilin 1, having an mP6-9 peptide inserted into each site of the L chain.

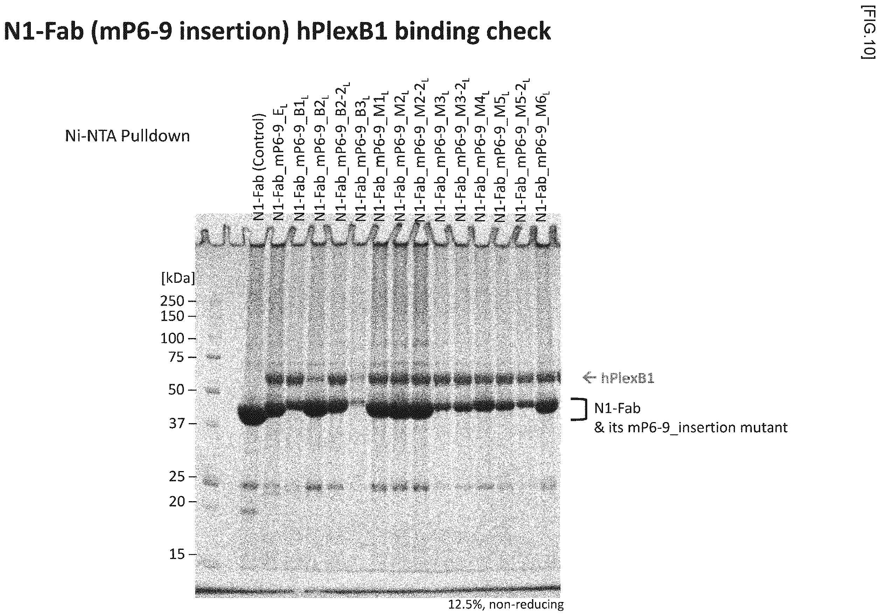

Fig. 10 shows the results of an Ni-NTA Pulldown test on the binding of the mP6-9 peptide fusion type N1_Fab to plexin B1 (hPIexB1).

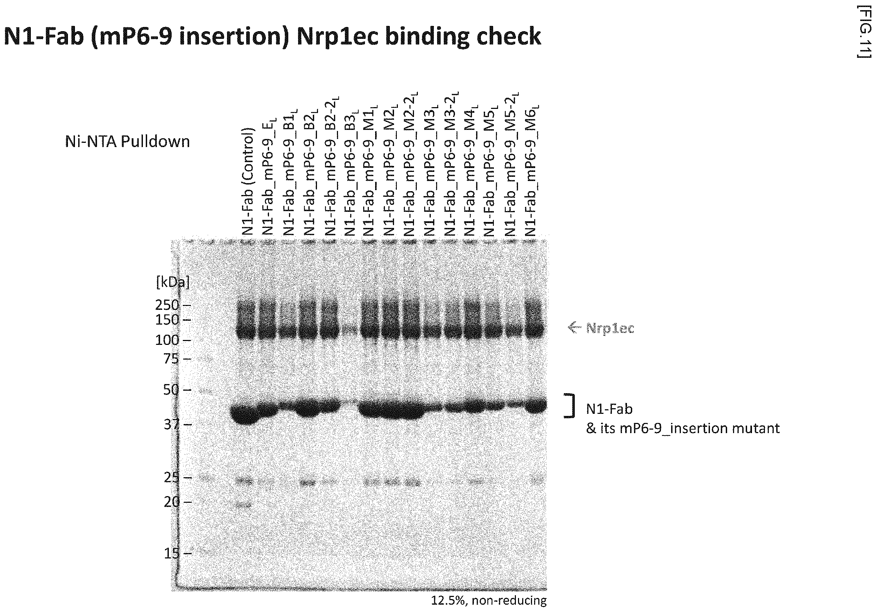

Fig. 11 shows the results of an Ni-NTA Pulldown test on the binding of the mP6-9 peptide fusion type N1_Fab to neuropilin 1 (Nrp1ec).

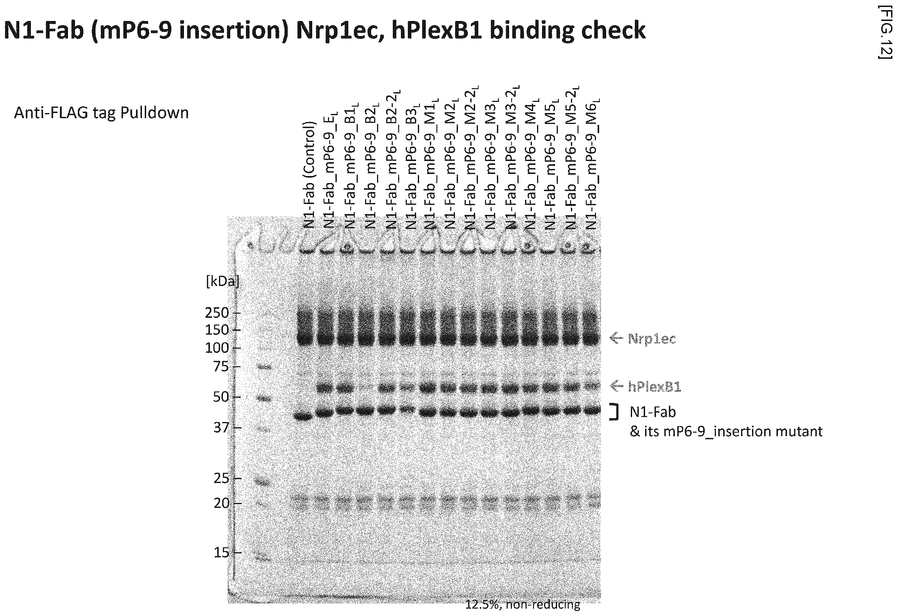

Fig. 12 shows the results of an Anti-FLAG tag Pulldown test on the binding of the P6-9 peptide fusion type N1_Fab to hPIexB1 and Nrp1ec.

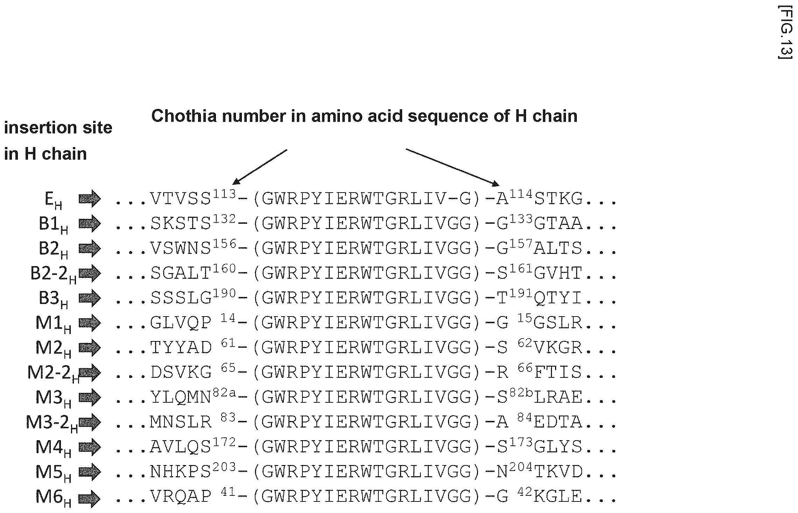

Fig. 13 shows an insertion site of a bioactive peptide in H chain. The number of an amino acid residue is according to numbering based on the Chothia amino acid residue number. The inserted bioactive peptide corresponds to the amino acid sequence of mP6-9.
Fig. 14 shows a steric structure of the Fab region of 1HZH, a human IgG antibody similar to that of Fig. 8. The structure placed on the nearer side shows an H chain and the structure placed on the far side shows an L chain. As insertion sites of a bioactive peptide in the H chain, shown are an elbow (E) site, B1 site, B2 site, B2-2 site, and B3 site.
Fig. 15 shows a steric structure of the Fab region of 1HZH, a human IgG antibody similar to that of Fig. 7. The structure placed on the nearer side shows an L chain and the structure placed on the far side shows an H chain. Shown as insertion sites of a bioactive peptide in the H chain are an M1 site, M2 site, M2-2 site, M3 site, M3-2 site, M4 site, M5 site, and M6 site.

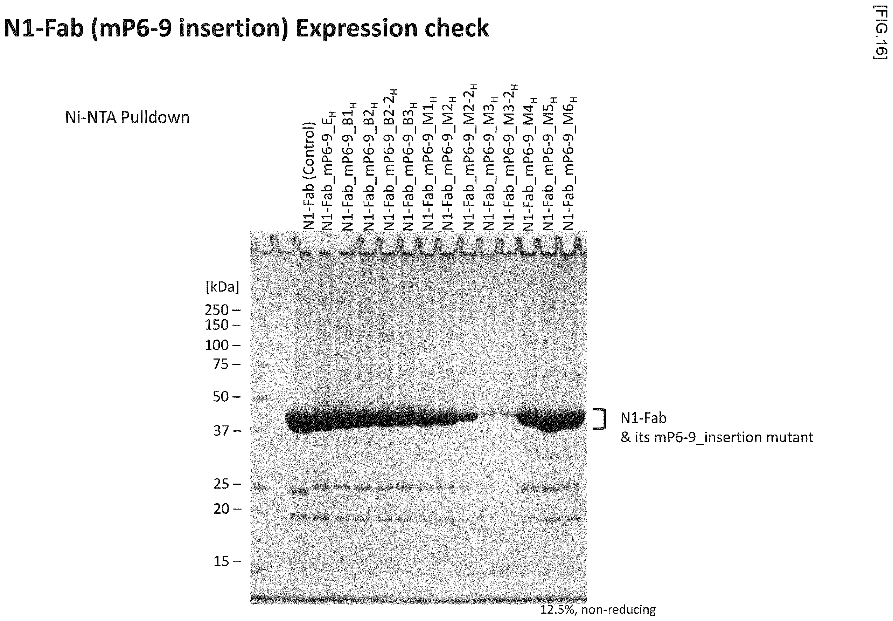

Fig. 16 shows the results of a Ni-NTA Pulldown test on the expression of the Fab (mP6-9 peptide fusion type N1_Fab) of a human IgG1 monoclonal antibody (N1 antibody, clone name: YW64.3) against neuropilin 1, having an mP6-9 peptide inserted into each site of the H chain.

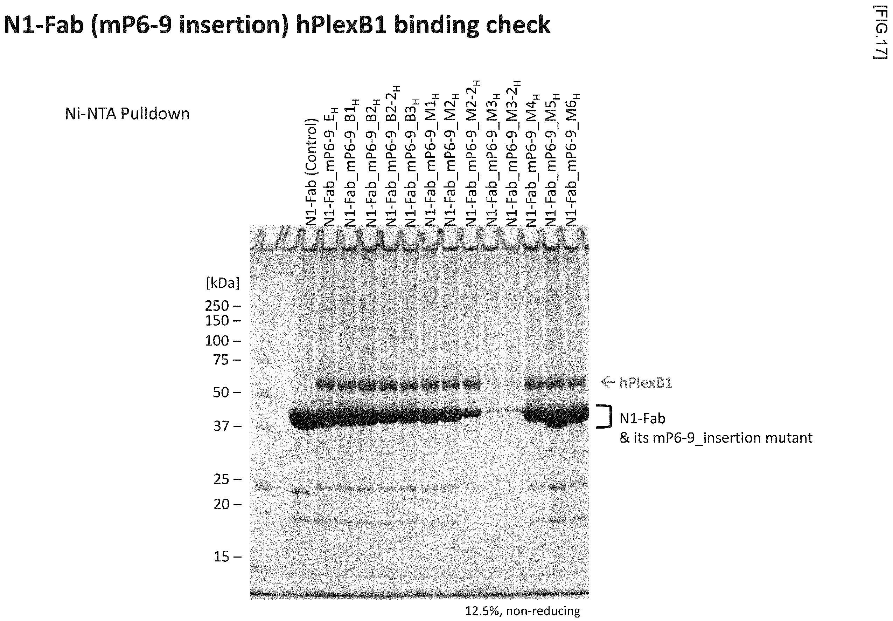

Fig. 17 shows the results of an Ni-NTA Pulldown test on the binding of the mP6-9 peptide fusion type N1_Fab to plexin B1 (hPIexB1).

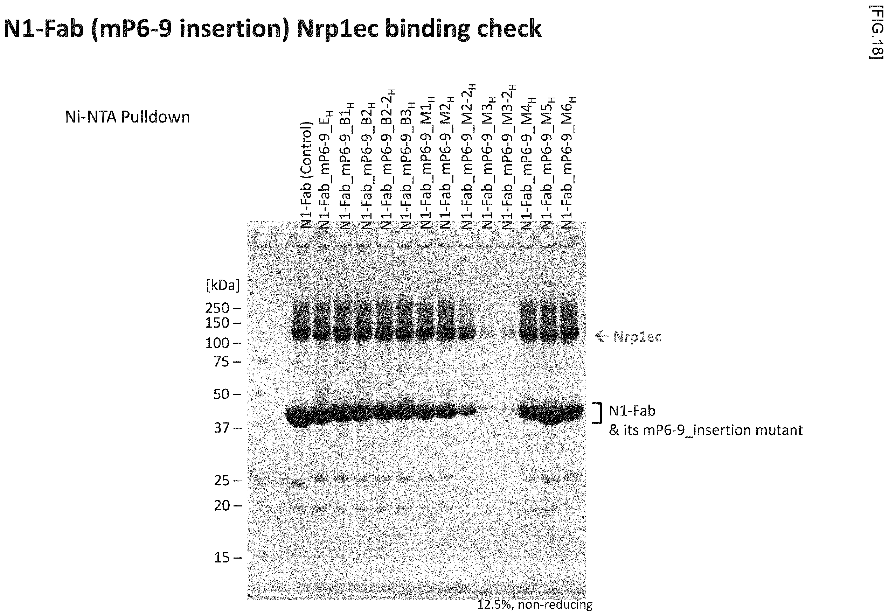

Fig. 18 shows the results of an Ni-NTA Pulldown test on the binding of the mP6-9 peptide fusion type N1_Fab to neuropilin 1 (Nrp1ec).

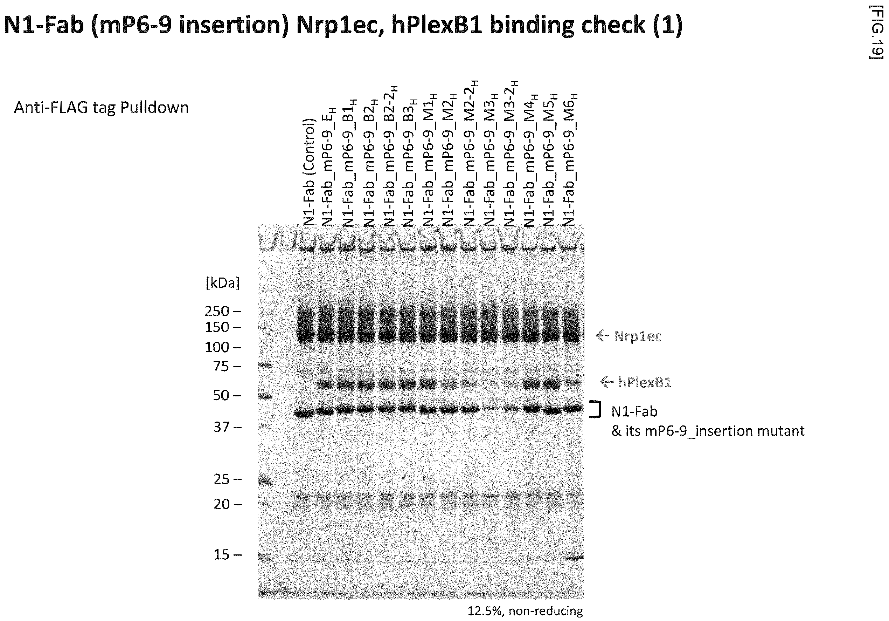

Fig. 19 shows the results of an Anti-FLAG tag Pulldown test on the binding of the P6-9 peptide fusion type N1_Fab to hPIexB1 and Nrp1ec.

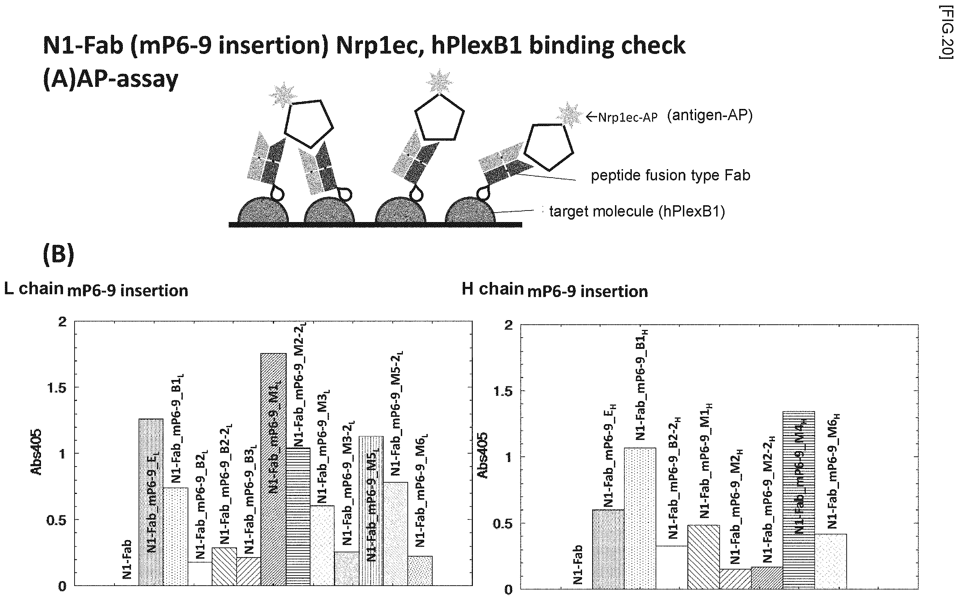

Fig. 20 shows a sandwich measurement system principle (A) showing the simultaneous binding of two target molecules and measurement results (B). Fig. 20A shows insertion of the bioactive peptide into a heavy chain.

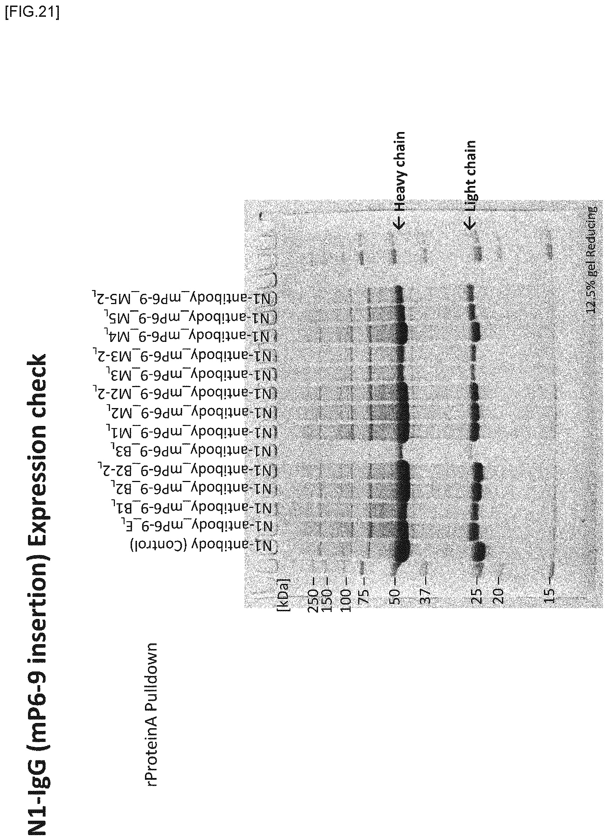

Fig. 21 shows the results of a rProteinA Pulldown test on the expression of a human IgG1 monoclonal antibody (N1 antibody, clone name: YW64.3) (mP6-9 peptide fusion type N1 antibody) against neuropilin 1, having an mP6-9 peptide inserted into each site of the L chain.

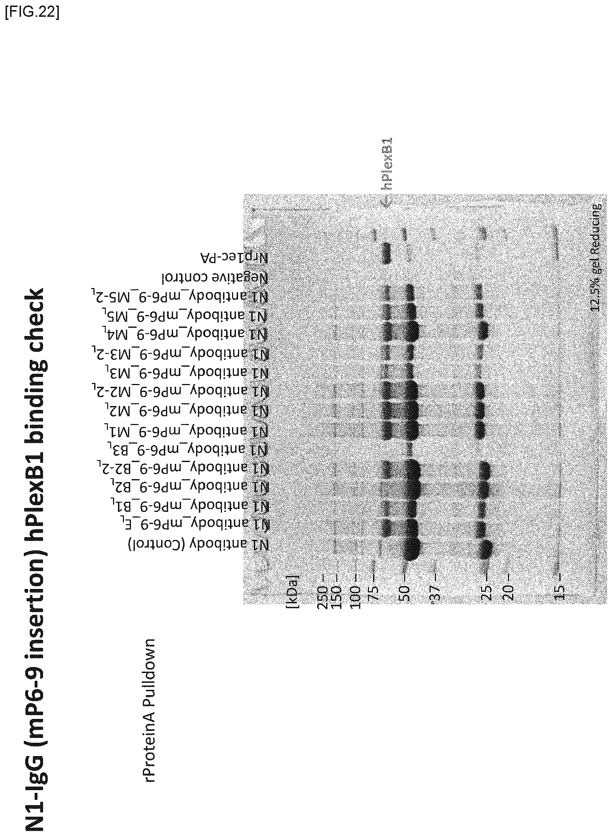

Fig. 22 shows the results of a rProteinA Pulldown test on the binding of the mP6-9 peptide fusion type N1 antibody to plexin B1 (hPIexB1).

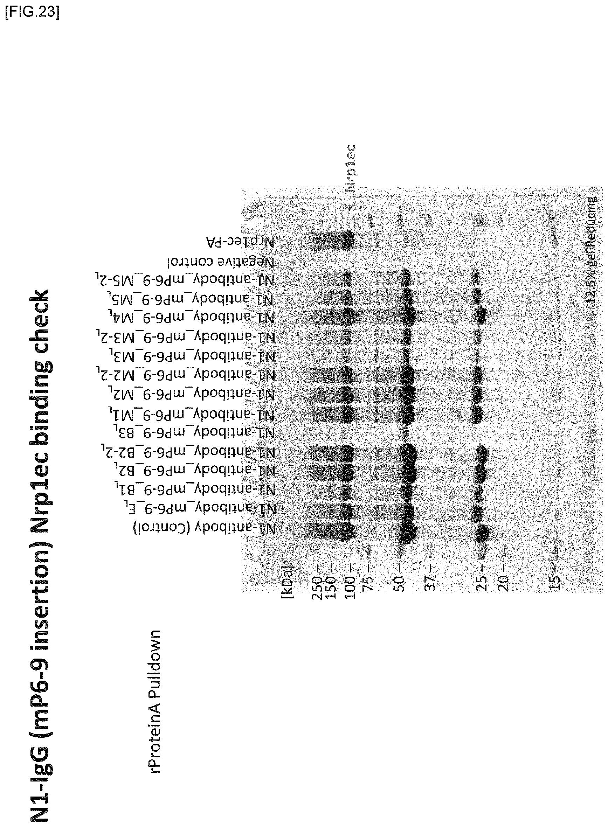

Fig. 23 shows the results of a rProteinA Pulldown test on the binding of the mP6-9 peptide fusion type N1 antibody to neuropilin 1 (Nrp1ec).

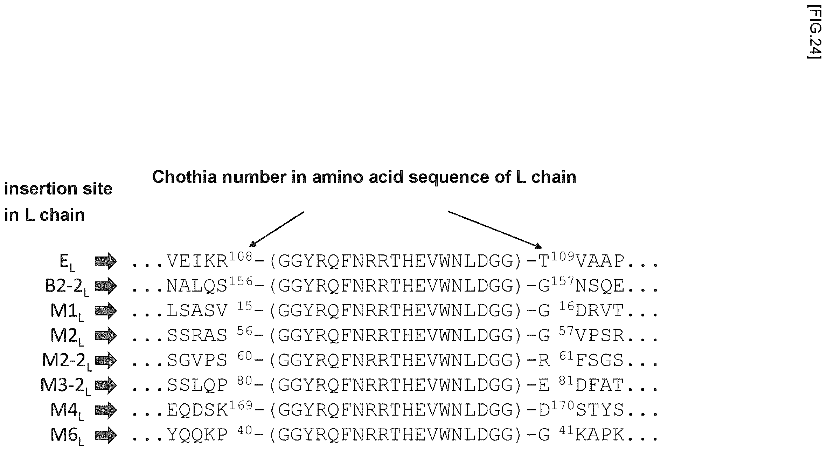

Fig. 24 shows the insertion site of a bioactive peptide in an L chain. The number of the amino acid residue is according to numbering based on the Chothia amino acid residue numbers. The inserted bioactive peptide corresponds to the amino acid sequence of aMD4.

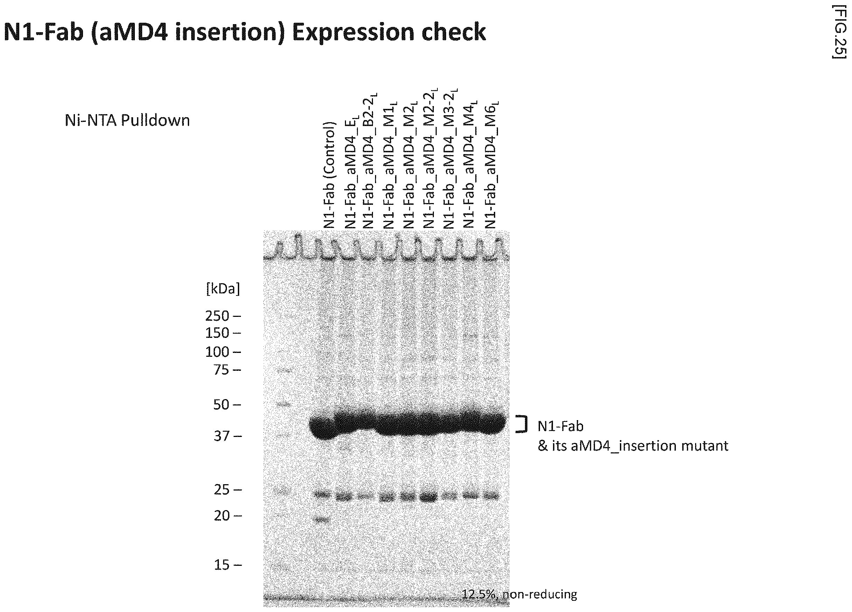

Fig. 25 shows the results of an Ni-NTA Pulldown test on the expression of the Fab (aMD4 peptide fusion type N1_Fab) of a human IgG1 monoclonal antibody (N1 antibody, clone name: YW64.3) against neuropilin 1, having an aMD4 peptide inserted into each site of the L chain.

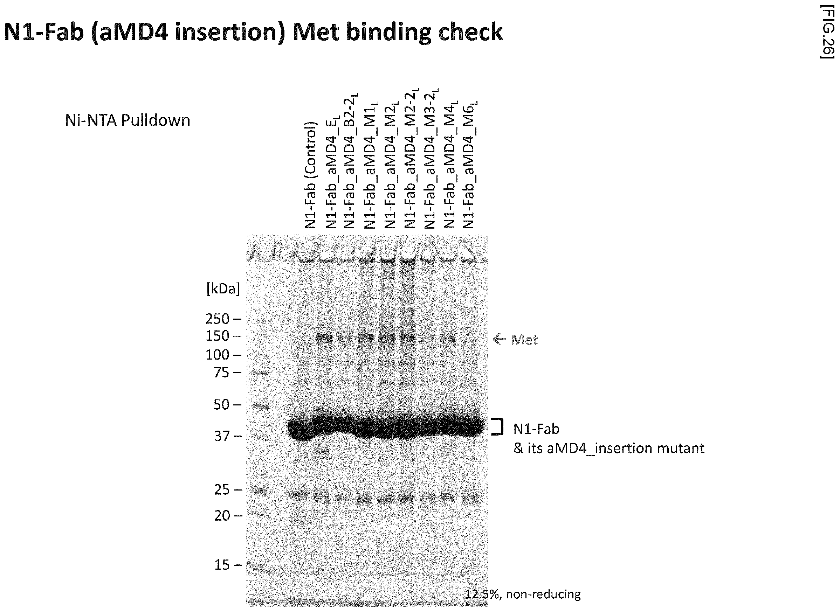

Fig. 26 shows the results of an Ni-NTA Pulldown test on the binding of the aMD4 peptide fusion type N1_Fab to a Met receptor (Met).

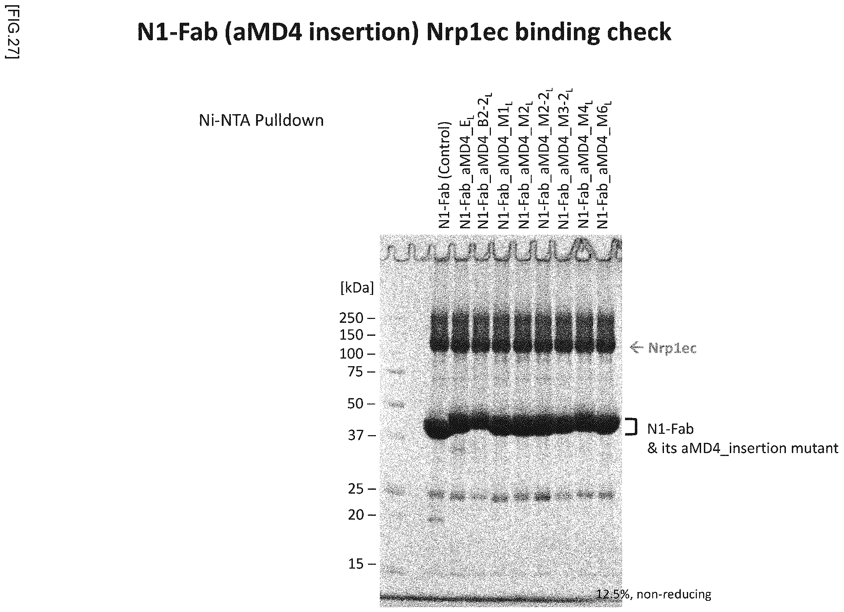

Fig. 27 shows the results of an Ni-NTA Pulldown test on the binding of the aMD4 peptide fusion type N1_Fab to neuropilin 1 (Nrp1ec).

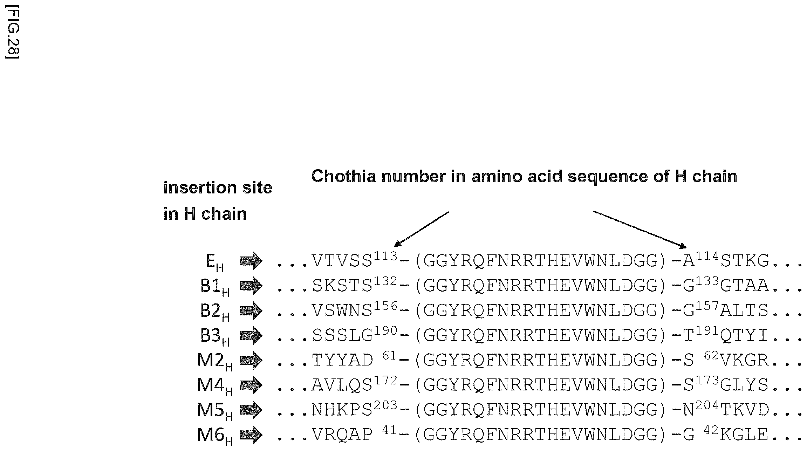

Fig. 28 shows the insertion site of a bioactive peptide in the H chain. The number of an amino acid residue is according to numbering based on the Chothia amino acid residue numbers. The inserted bioactive peptide corresponds to the amino acid sequence of aMD4.

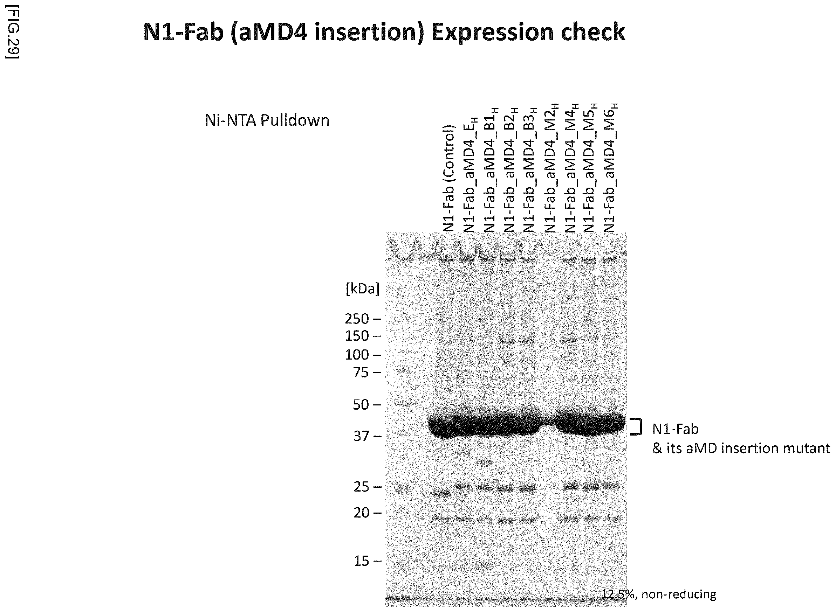

Fig. 29 shows the results of an Ni-NTA Pulldown test on the expression of the Fab (aMD4 peptide fusion type N1_Fab) of a human IgG1 monoclonal antibody (N1 antibody, clone name: YW64.3) against neuropilin 1, having an aMD4 peptide inserted into each site of the H chain.

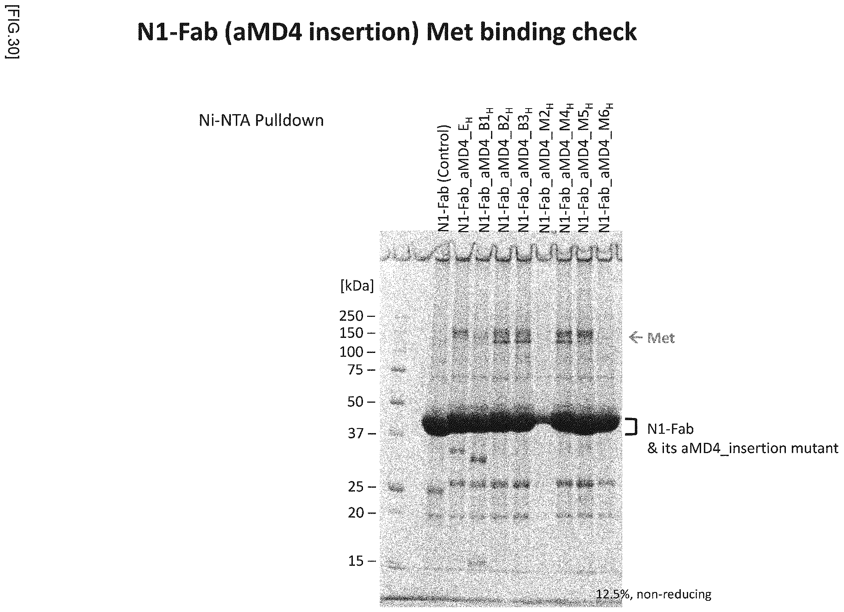

Fig. 30 shows the results of a Ni-NTA Pulldown test on the binding of the aMD4 peptide fusion type N1_Fab to a Met receptor (Met).

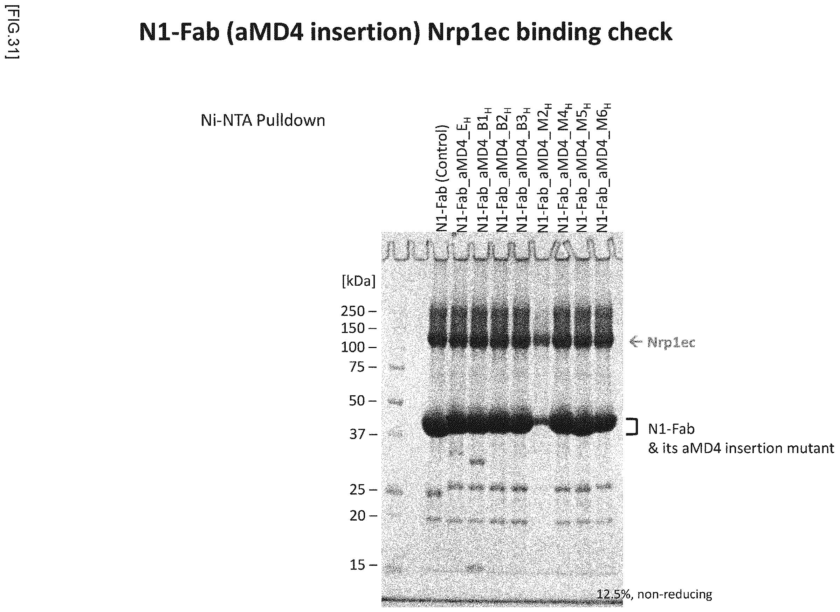

Fig. 31 shows the results of a Ni-NTA Pulldown test on the binding of the aMD4 peptide fusion type N1_Fab to neuropilin 1 (Nrp1ec).

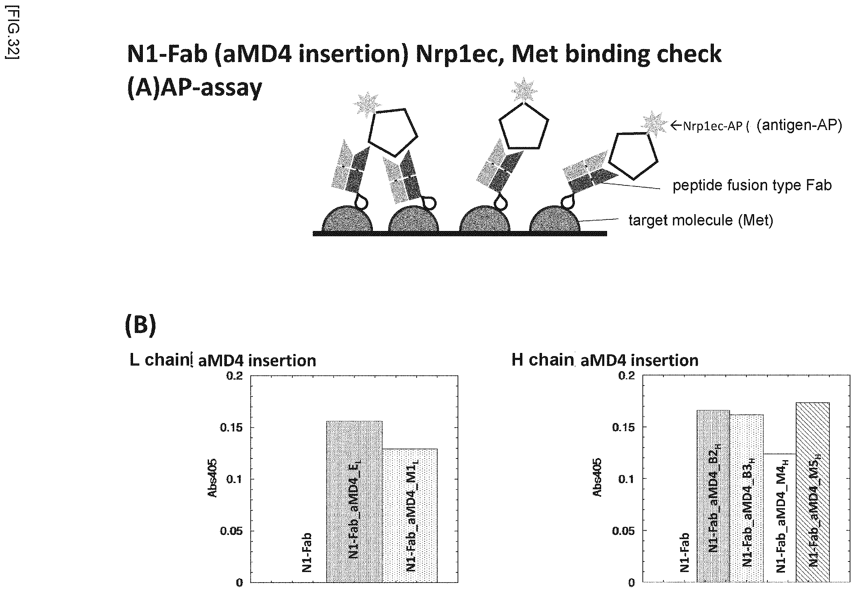

Fig. 32 shows the principle (A) of a sandwich measurement system showing simultaneous binding of two kinds of target molecules and measurement results (B). Fig. 32A shows insertion of a bioactive peptide into a heavy chain.

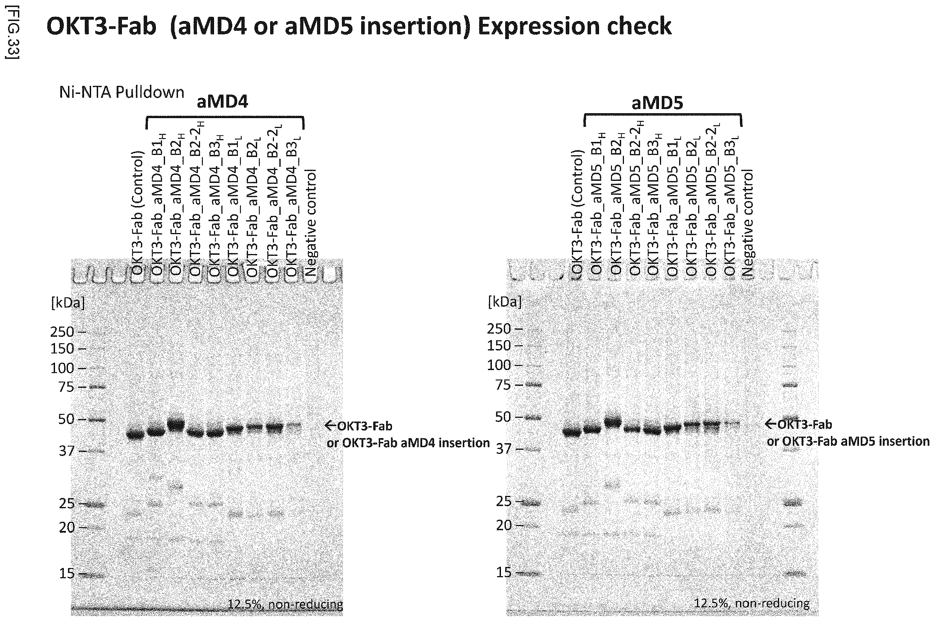

Fig. 33 shows the results of a Ni-NTA Pulldown test on the expression of the Fab (aMD4 peptide fusion type OKT3_Fab or aMD5 peptide fusion type OKT3_Fab) of an anti-CD3 antibody (OKT3) having an aMD4 peptide or an aMD5 peptide inserted into each site of the L chain or H chain.

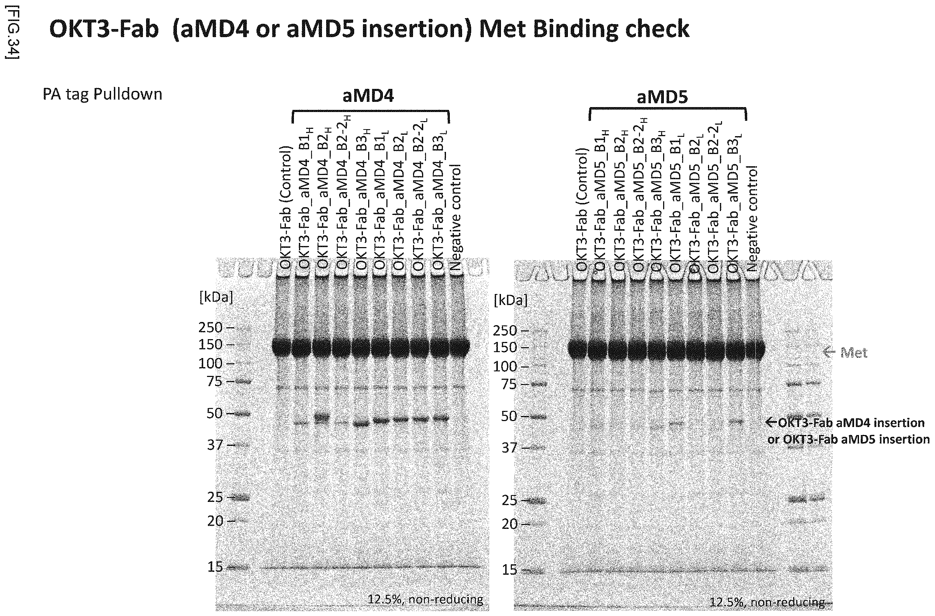

Fig. 34 shows the results of a PA tag Pulldown test on the binding of the aMD4 or aMD5 peptide fusion type OKT3_Fab to a Met receptor (Met).

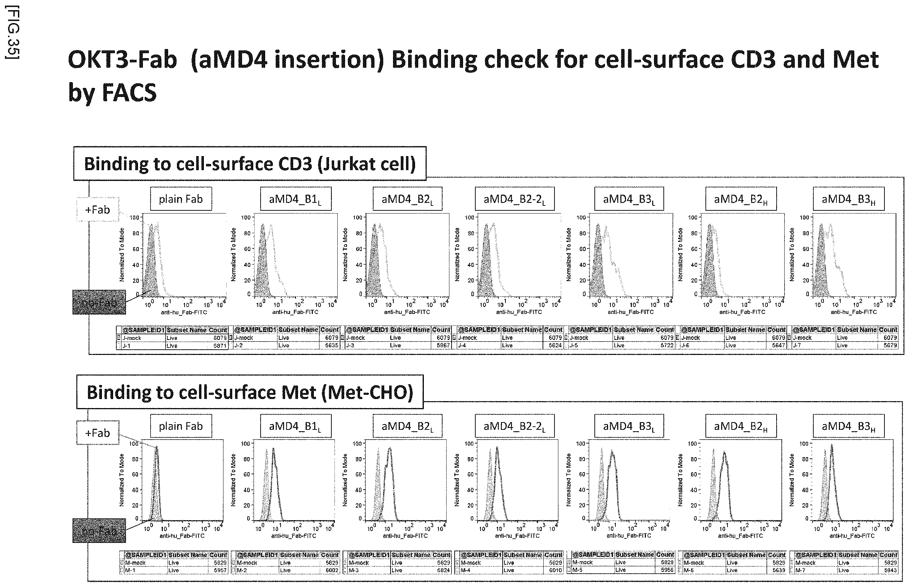

Fig. 35 shows the results of evaluation, by FACS, of the binding of the aMD4 peptide fusion type OKT3_Fab to CD3 or Met expression cells.

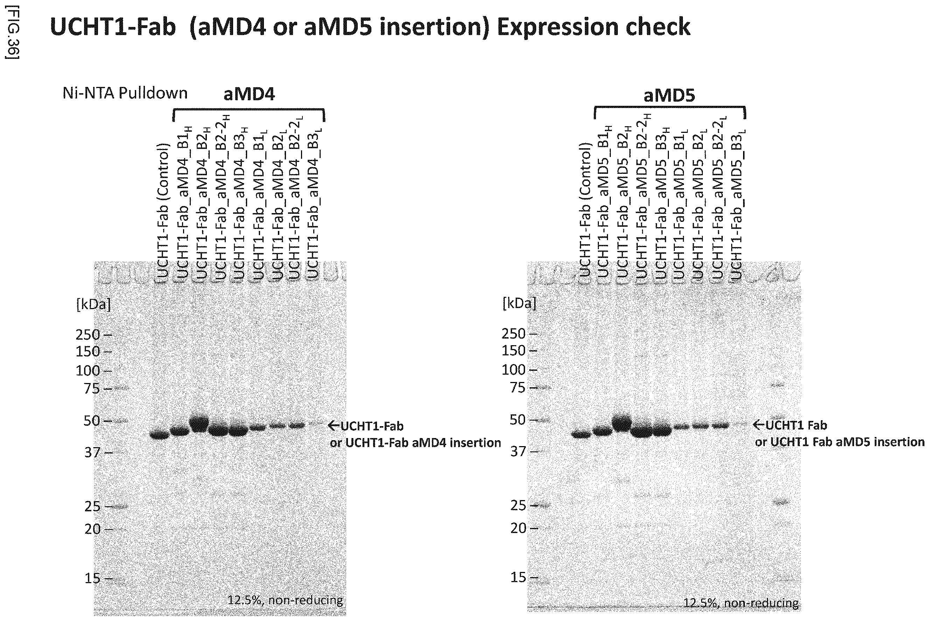

Fig. 36 shows the results of a Ni-NTA Pulldown test on the expression of the Fab (aMD4 peptide fusion type UCHT1_Fab or aMD5 peptide fusion type UCHT1_Fab) of an anti-CD3 antibody (UCHT1) having an aMD4 peptide or aMD5 peptide inserted into each site of the L chain or H chain.

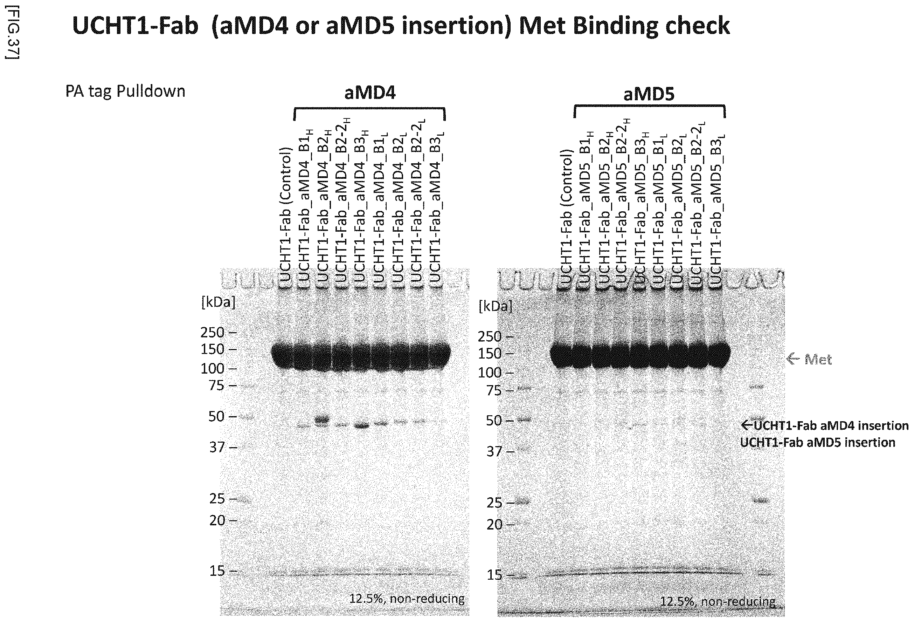

Fig. 37 shows the results of a PA tag Pulldown test on the binding of the aMD4 or aMD5 peptide fusion type UCHT1_Fab to a Met receptor (Met).

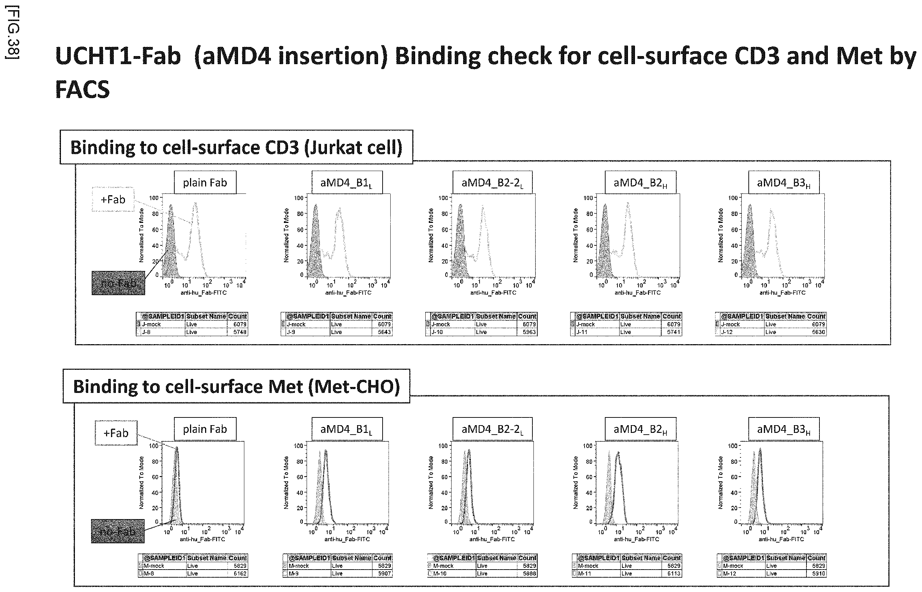

Fig. 38 shows the results of evaluation, by FACS, of binding of the aMD4 or aMD5 peptide fusion type UCHT1_Fab to CD3 or Met expression cells.

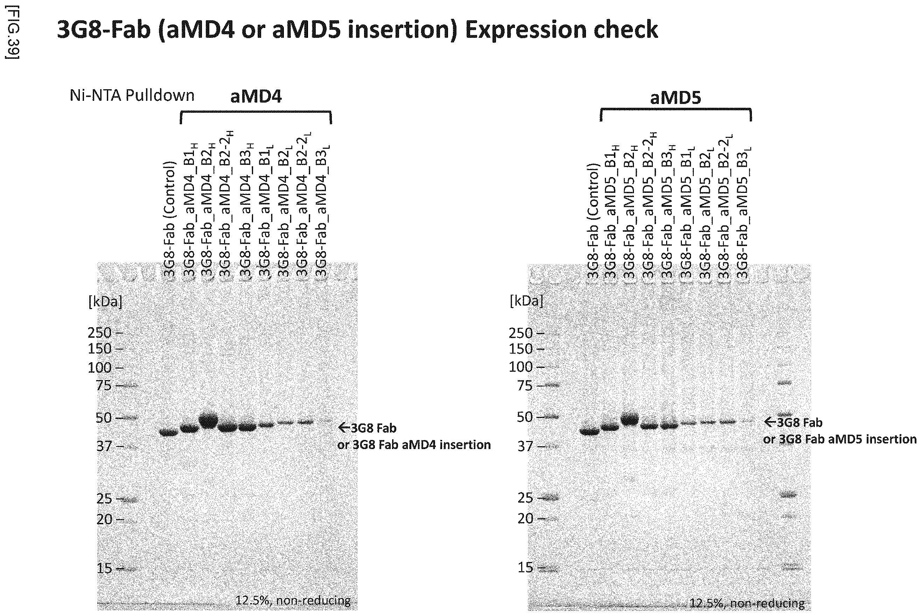

Fig. 39 shows the results of a Ni-NTA Pulldown test on the expression of the Fab (aMD4 peptide fusion type 3G8_Fab or aMD5 peptide fusion type 3G8_Fab) of an anti-CD16 antibody (3G8), having an aMD4 peptide or aMD5 peptide inserted into each site of the L chain or H chain.

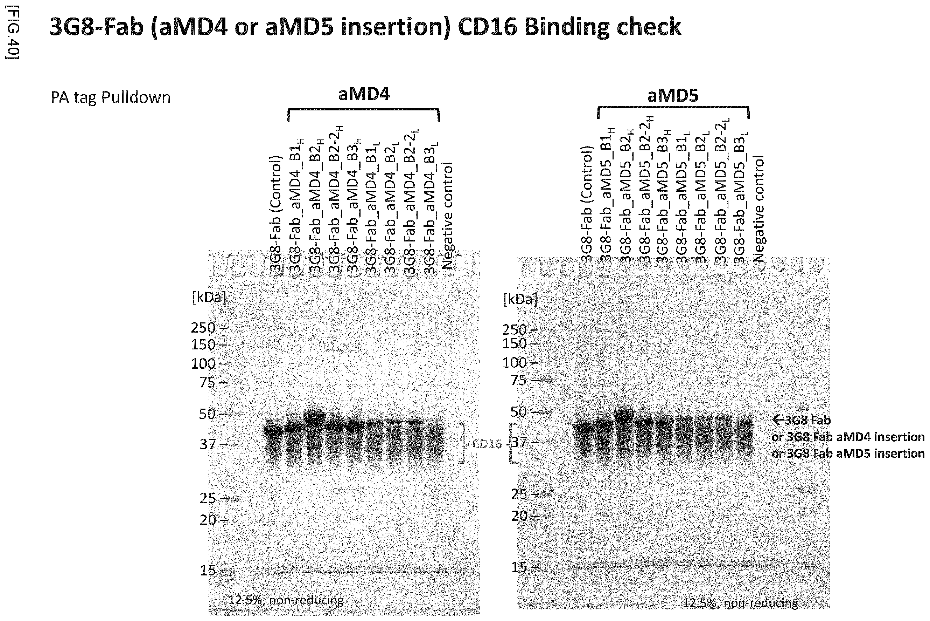

Fig. 40 shows the results of a PA tag Pulldown test on the binding of the aMD4 or aMD5 peptide fusion type 3G8_Fab to a CD16 receptor (CD16).

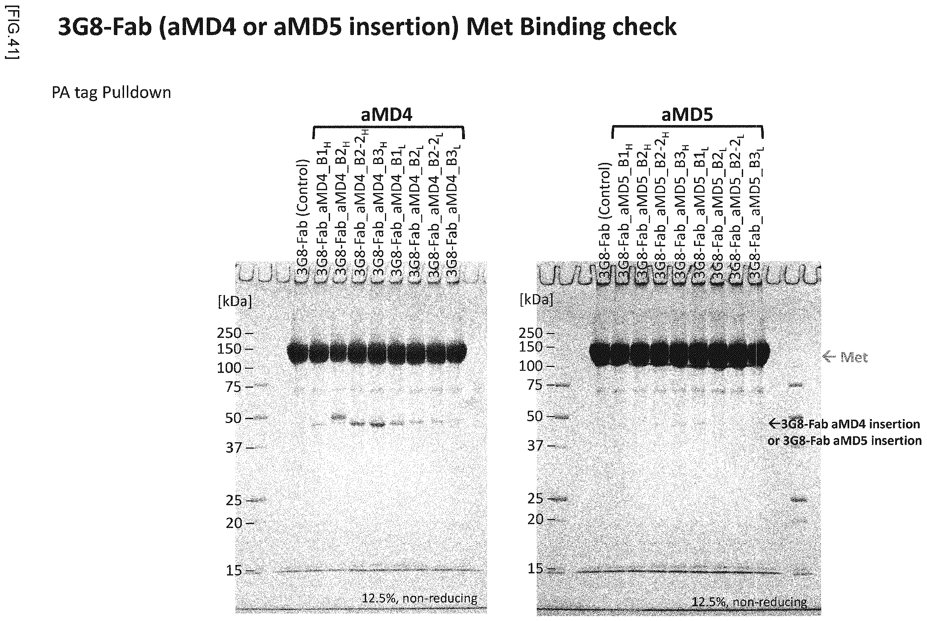

Fig. 41 shows the results of a PA tag Pulldown test on the binding of the aMD4 or aMD5 peptide fusion type 3G8_Fab to a Met receptor (Met).

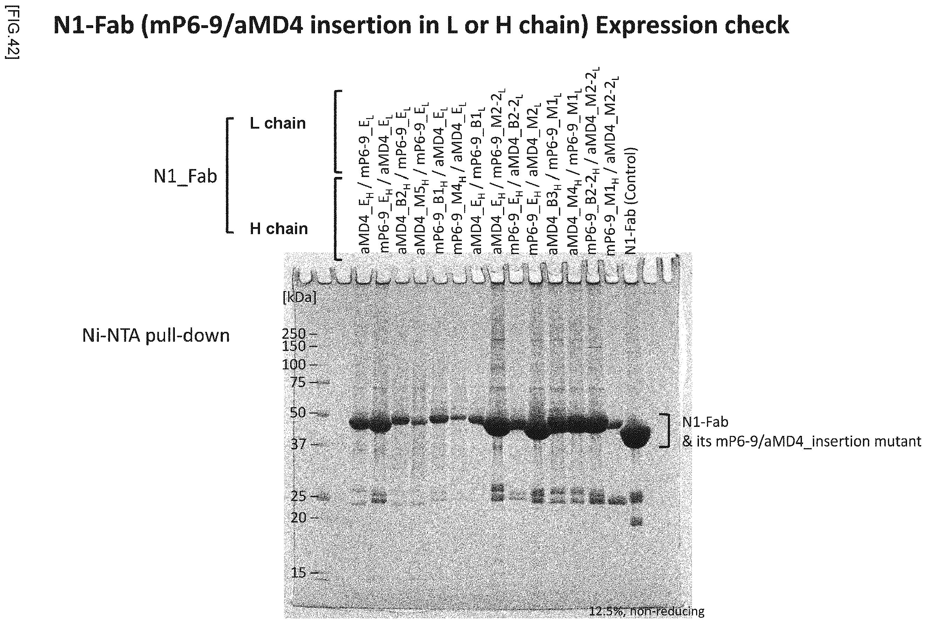

Fig. 42 shows the results of a Ni-NTA Pulldown test on the expression of the Fab (trispecific Fab) of a human IgG1 monoclonal antibody (N1, clone name: YW64.3) against neuropilin 1, having a mP6-9 peptide and an aMD4 peptide inserted into each site of the L chain or H chain.

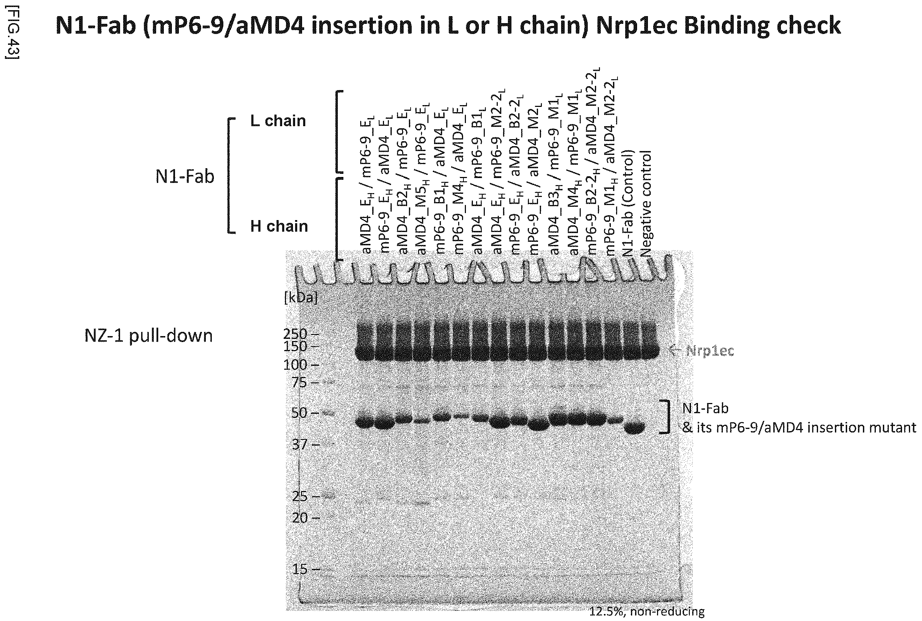

Fig. 43 shows the results of a NZ-1 Pulldown test on the binding of the trispecific Fab to neuropilin 1 (Nrp1ec).

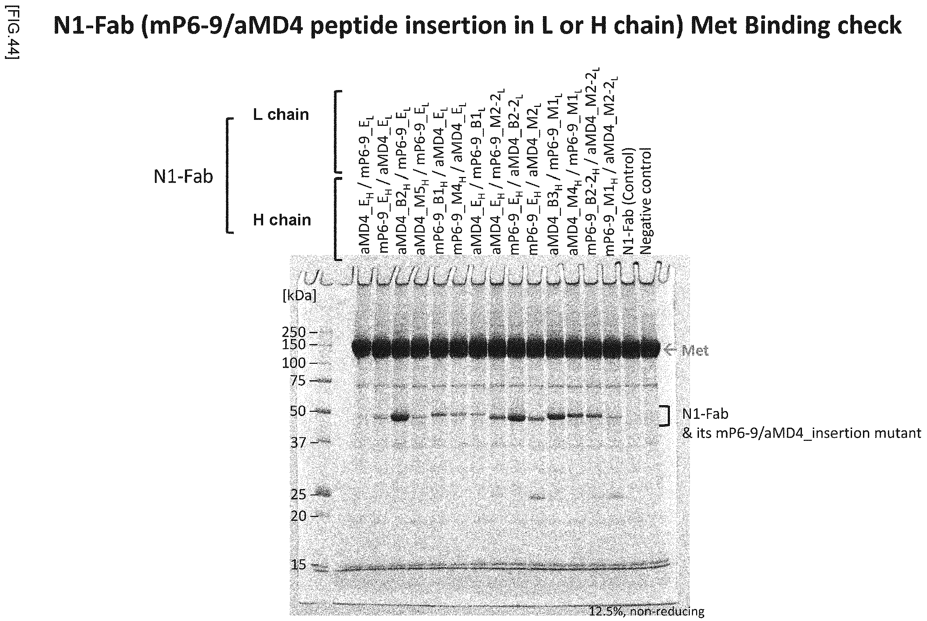

Fig. 44 shows the results of binding of the trispecific Fab to plexin B1 (hPIexB1).

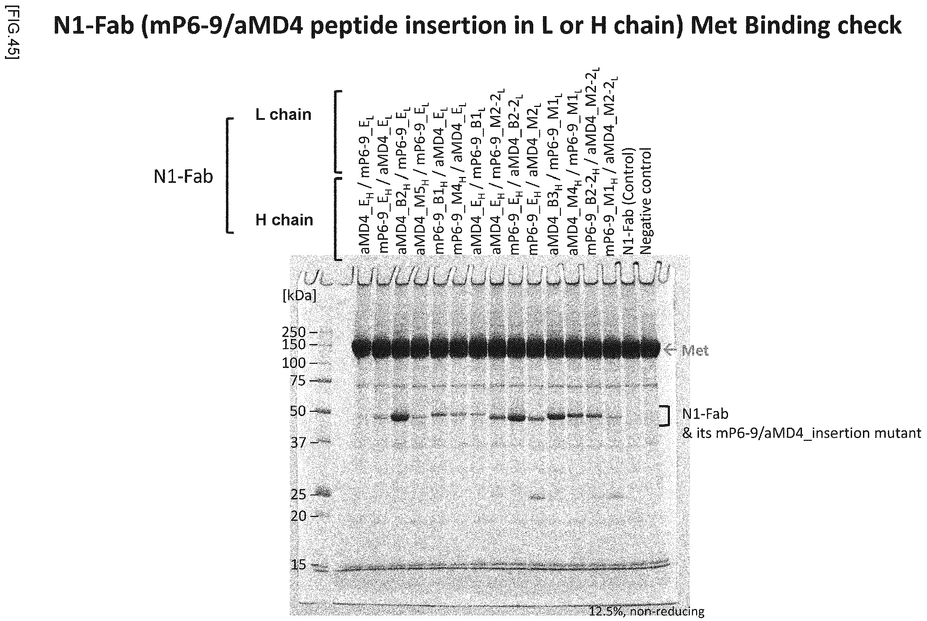

Fig. 45 shows the results of binding of the trispecific Fab to a Met receptor (Met).

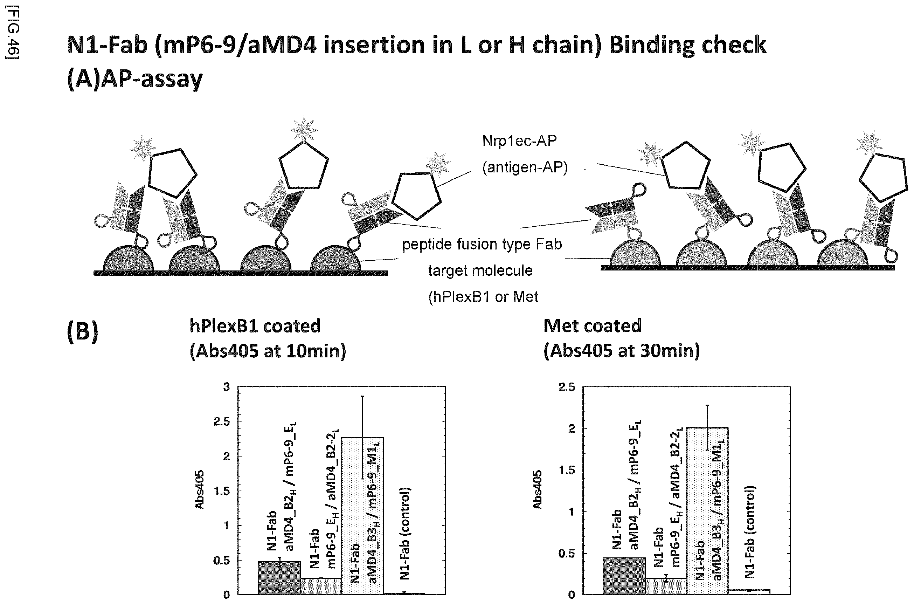

Fig. 46 shows the principle (A) of a sandwich measurement system showing simultaneous binding of two kinds of target molecules to the trispecific Fab and measurement results (B).

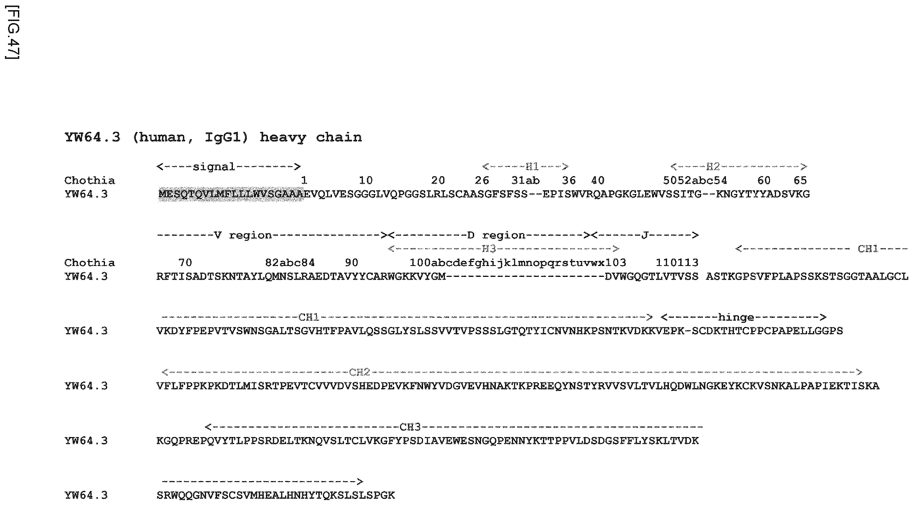

Fig. 47 shows the amino acid sequence of the H chain of a human IgG1 monoclonal antibody (N1 antibody, clone name: YW64.3) against neuropilin 1.

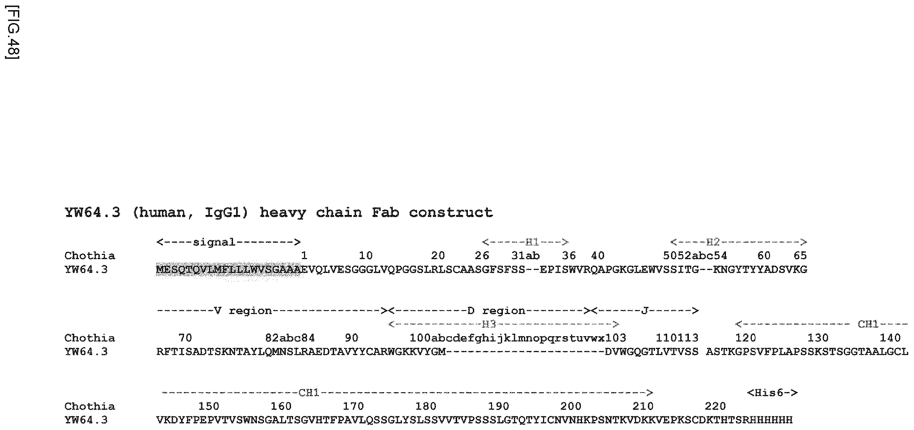

Fig. 48 shows the amino acid sequence of a N1 antibody H-chain Fab region produced from the antibody H-chain Fab region expression vector described in Example.

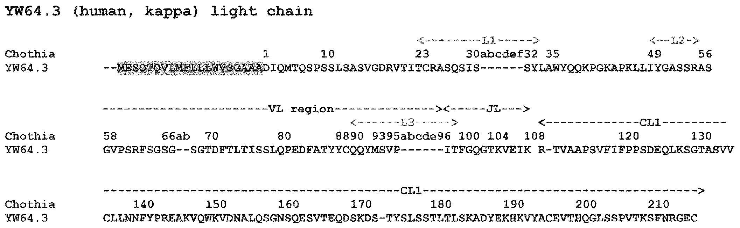

Fig. 49 shows the amino acid sequence of a N1 antibody L_{K} chain produced from the antibody L_{K} chain expression vector described in Example.

### Description of Embodiments

The present invention will be described more specifically by modes for carrying out the invention. The present invention is not limited by the following modes for carrying out the present invention, but can be carried out after various modifications.

### [Artificial protein]

The artificial protein of the present invention has a bioactive peptide fused with a site, of a protein having at least an antigen-binding region of an antibody, present in a folded portion of a secondary structure in a structural region having an antigen-binding activity and exposed on the surface of the protein.

The artificial protein of the present invention has an antigen-binding region of an antibody. The artificial protein of the present invention has an antigen-binding region so that the artificial protein retains a structure having an antigen-binding activity as the structure of the artificial protein, based on the antigen binding region.

The artificial protein of the present invention has, in the structural region having an antigen-binding activity, a folded portion of a secondary structure and this folded portion has therein a site exposed on the surface of the protein as a steric structure of the protein.

In the present invention, a bioactive peptide is fused with the site exposed on the surface of the protein.

All of the folded portions of the secondary structure may be exposed on the surface of the protein or some of the folded portions of the secondary structure may be exposed on the surface of the protein.

The antibody usually has two tip portions made of 6 CDRs present in H and L chains. In the present invention, examples of the antigen-binding region of an antibody which the protein at least has include a tip portion made of 6 CDRs in H and L chains. Although an antibody has two tip portions, the artificial protein of the present invention may have one such tip portion.

Examples of the protein having such a tip portion include antibodies. Although the antibodies are not particularly limited, they can be classified into five kinds, that is, IgG, IgM, IgA, IgD, and IgE.

When the protein having at least an antigen-binding region of an antibody is an antibody in the present invention, any of IgG, IgM, IgA, IgD, and IgE may be used as an antibody, but IgG is preferred. A number of subclasses are known as a subclass of IgG and it may be selected from them.

As the antibody, no particular limitation is imposed on what the antibody is derived from and it may be a mouse antibody, a rat antibody, a rabbit antibody, a horse antibody, a canine antibody, a chimeric antibody, a humanized antibody, or a complete human antibody.

The protein having at least an antigen-binding region of an antibody may be a fragment of the antibody as described above and examples include Fv, Fab, scFv, Diabody, and taFv.

It is to be noted that the protein having at least an antigen-binding region of an antibody may be a structure shown in FIGS. 2 to 5.

The Fv, Fab, scFv, Diabody, or taFv can be an antibody fragment which can be understood as a structure already known in the prior art.

The artificial protein of the present invention has a bioactive peptide fused in a structural region having an antigen-binding activity and the artificial protein preferably retains the antigen-binding activity which the protein has before being fused with the bioactive peptide.

The term "artificial protein retains the antigen-binding activity" as used herein means that even after the artificial protein is fused with a bioactive peptide, the resulting artificial protein retains the antigen-binding activity at the time when it was an intact protein. In this case, the antigen-binding activity may be different in degree before and after the bioactive peptide is fused.

In the present invention, the antigen-binding activity is preferably an antigen-binding activity of an antibody when a protein having an antigen-binding region of an antibody is regarded as the antibody.

The artificial protein of the present invention fused with a bioactive peptide has preferably a function derived from the bioactive peptide.

The artificial protein of the present invention preferably retains its antigen-binding activity and has a function derived from the bioactive peptide thus fused. When the artificial protein of the present invention retains its antigen-binding activity and has a function derived from the bioactive peptide thus fused, the artificial protein of the present invention can be regarded as an artificial protein having multispecificity.

As a preferred mode, the present invention can provide an engineering technology of an authentic antibody itself including giving second binding specificity to the periphery of a Fv region via a bioactive peptide to be fused.

The term "Fv region" as used herein means an independent structural region composed of the VH domain of an H chain and the VL domain of an L chain.

In the present invention, the artificial protein having an antigen-binding region of an antibody may be an artificial protein having, in addition to the function derived from the antigen-binding region of the antibody, a new function because it has a bioactive peptide fused with a site present in a folded portion of a secondary structure in a structural region having an antigen-binding activity and exposed on the surface of the protein. More specifically, in a preferred mode, the artificial protein is an antibody-like protein having a new function derived from a bioactive peptide, because the artificial protein having at least an antigen-binding region of an antibody exhibits an antigen-binding property which the antibody essentially has and moreover, it has a bioactive peptide fused in the vicinity of the antigen-binding region.

Accordingly, when the artificial protein of the present invention exhibits the antigen-binding property which the antibody essentially has and further exhibits a new function derived from the bioactive peptide fused therewith, it is an artificial protein having at least double specificity.

Preferably, the artificial protein of the present invention retains an antigen-binding activity derived from the antibody and has a function derived from the bioactive peptide fused therewith and thus has multispecificity. The artificial protein of the present invention can have a dual antigen-binding property which the antibody essentially has and/or the artificial protein of the present invention can have multispecificity because it has a plurality of bioactive peptides fused therewith and two or more respectively different bioactive peptides exhibit two or more respectively different bioactivities.

In protein engineering, in order to impart a certain protein including an antibody with a certain binding activity, it is the common practice to use a method of creating a library based on the protein by randomizing a plurality of loops close to each other in the protein and acquiring a product bound to a desired target molecule from the library. Also in Fcab, a method of randomizing loops at two or three positions is employed.

The present invention is advantageous in that a bioactive peptide can be fused with a protein by fusing the bioactive peptide with a site present in a folded portion of a secondary structure in a structural region having an antigen-binding activity and exposed on the surface of the protein, without using the prior art method.

In the artificial protein of the present invention, the structure fused with a site present in a folded portion of a secondary structure in a structural region having an antigen-binding activity and exposed on the surface of the protein can be preferably only an amino acid structure derived from the bioactive peptide.

### [Bioactive peptide]

Although the bioactive peptide to be fused with the artificial protein is not particularly limited, it is preferably a cyclic peptide or a linear peptide.

The term "bioactive peptide" means a peptide having a certain bioactivity and the biological activity is not particularly limited.

The bioactivity of the bioactive peptide is preferably a binding activity to a desired molecule, more preferably a binding activity to a desired target molecule. The binding activity may activate the activity of a molecule by binding thereto, inhibit the activity of a molecule, or may control the activity of a molecule.

The bioactive peptide may be an inhibitor, an activator, or a peptide classified into antagonist or an agonist. The bioactive peptide can also be said to be a peptide showing a binding activity to a certain target molecule by the bioactive peptide alone.

The target molecule is not particularly limited, but it is preferably a molecule which becomes a target in medicaments and examples of it include receptors and enzymes.

With regard to the intensity of the bioactivity, the bioactive peptide may have an activity in the order of pM, activity in the order of nM, activity in the order of µM, or an activity in the order of mM when expressed as a Kd value or EC₅₀ value, but it has preferably an activity in the order of nM or less.

With regard to the bioactivity, the bioactive peptide may have an enhanced or attenuated activity by being fused with the artificial protein.

When the bioactive peptide is fused with the artificial protein of the present invention, the full length of the amino acid sequence known to have a bioactivity may be fused with the artificial protein or a partial sequence of it may be fused therewith.

When the partial sequence of the bioactive peptide is fused, the partial sequence is selected preferably to retain the bioactivity of the bioactive peptide.

When the bioactive peptide is fused as the partial sequence, it may be fused so as to retain its bioactivity by presenting it on the surface of the artificial protein. In other words, a bioactive peptide having a partial sequence and having a weaker bioactivity than the full-length peptide is also a preferred mode in the present invention when it is fused with the artificial protein to retain a bioactivity which the bioactive peptide essentially has.

When the bioactive peptide is a cyclic peptide, it is preferably fused with the artificial protein as a chain peptide obtained by cleaving a specific bond in the cyclic structure of the cyclic peptide.

The cleavage site of the specific bond in the cyclic peptide is preferably a site retaining a bioactivity after fusion of the cleavage site with the protein.

The full length of the amino acid sequence when the full length of the cyclic peptide is expressed as a chain peptide may be fused with the artificial protein or the partial sequence of the amino acid sequence may be fused with the artificial protein.

In other words, the bioactive peptide having a binding activity to a desired molecule is preferably fused while retaining the binding activity to the desired molecule.

When the bioactive peptide is inserted into the artificial protein, the amino acid portion derived from the artificial protein may be linked directly to the amino acid portion derived from the bioactive peptide or they may be linked to each other via a certain linker.

Although the linker is not particularly limited insofar as it can link the bioactive peptide to the original protein having at least an antigen-binding region of an antibody, it has preferably a bioinactive amino acid sequence.

The linker has preferably a sequence having one or two or more amino acids such as glycine and serine linked to each other.

The bioactive peptide is not particularly limited and for example, it is a peptide having from 5 to 20 amino acids.

When the bioactive peptide is fused, it may have all the amino acids of the bioactive peptide or one or more amino acids may be substituted, deleted, or inserted within such a range as retaining a bioactivity.

The term "one or more" may be 1, 2, 3, 4, 5, 6, 7, 8, 9, or 10, within a range of from 1 to 10, or from 1 to 9, from 1 to 8, from 1 to 7, from 1 to 6, from 1 to 5, from 1 to 4, from 1 to 3, 1 or 2, or 1.

When the bioactive peptide is fused as a mutant thereof, the mutant may be fused as an amino acid sequence having sequence homology of 70% or more, 80% or more, 85% or more, 90% or more, or 95% or more with the amino acid sequence which the original bioactive peptide has, insofar as the bioactivity does not disappear.

When the bioactive peptide is a cyclic peptide, it may be a naturally occurring cyclic peptide, a peptide produced by a common mRNA display method, a peptide produced by a TRAP method or a RaPID method, or a peptide produced by a phage display method. Alternatively, it may be a peptide produced by a modification thereof.

The cyclic peptide contains, for example, a thioether bond or a disulfide bond as a chemical crosslinking structure for forming an intramolecular cyclic structure.

Usually, in the cyclic peptide selected by an mRNA display method such as RaPID method or TRAP method or a phage display method, a structure, other than the chemical crosslinking structure for forming an intramolecular cyclic structure, such as thioether bond or disulfide bond is often an active site having a bioactivity.

Then, it is preferred that an apparent linear peptide obtained by cleaving a thioether bond or disulfide bond of a cyclic peptide is supposed and the resulting linear peptide is fused with the artificial protein. In this case, high specificity and affinity of the cyclic peptide obtained by an mRNA display method such as RaPID method or TRAP method or a phage display method can usually be given into a desired structure of a desired artificial protein. Although not particularly limited, a cyclic peptide available by an mRNA display method such as RaPID method or TRAP method or a phage display method can be fused with versatility by fusing a cyclic peptide having a thioether bond or a disulfide bond as an intramolecular cyclic structure.

Although the cyclic peptide is not particularly limited, a naturally occurring cyclic peptide may be used or a non-naturally occurring peptide may be used.

In order to fuse a naturally occurring cyclic peptide onto the artificial protein, any bonding can be employed because any bonding for bonding amino acid residues to each other is regarded as the chemical crosslinking structure for forming an intramolecular cyclic structure. It is however preferred that the bonding of amino acid residues in a region other than a region of the cyclic peptide considered as an active site is preferably formed as a chemical crosslinking structure for fusing with the artificial protein.

The cyclic peptide is a peptide having, in the molecule thereof, at least a cyclic structure composed of four or more amino acid residues. The cyclic structure in the cyclic peptide composed of four or more amino acid residues is a ring-closed structure of a linear peptide formed in the molecule thereof by bonding two amino acid residues, which are separated from each other by two or more amino acids, directly or via a linker or the like.

The term "two amino acid residues separated by two or more amino acids" has the same meaning as presence of at least two amino acid residues between two amino acid residues. Two amino acid residues are bound to each other with two or more amino acids therebetween.

In the present invention, a bioactive peptide is fused with a site present in a folded portion of a secondary structure in a structural region having an antigen-binding activity and exposed on the surface of a protein, but the number of amino acids constituting the bioactive peptide thus fused is not particularly limited.

It is preferred that the artificial protein of the present invention retains an antigen-binding activity so that fusion of the bioactive peptide enough to deprive the structural region having the antigen-binding activity of the antigen-binding activity is not desired.

The lower limit of the number of the amino acids of the bioactive peptide is not particularly limited but it may be 4, while the upper limit is not particularly limited but it may be 30 or less, 25 or less, or 20 or less.

In the artificial protein of the present invention, the number of amino acids may increase or decrease after the fusion of the bioactive peptide.

### [Protein having at least an antigen-binding region of an antibody]

The artificial protein of the present invention may also be a fusion protein obtained by inserting a bioactive peptide into a protein having at least an antigen-binding region of an antibody. Typical examples of the protein having at least an antigen-binding region of an antibody include proteins shown in Figs. 2 to 5. It is preferably Fv, Fab, scFv, IgG, Diabody, or taFv, more preferably Fab, scFv, or IgG.

Accordingly, the antigen-binding activity based on the antigen binding region of an antibody is preferably an antigen-binding activity of an antibody or a derivative thereof in a structure such as Fv, Fab, scFv, IgG, Diabody, or taFv before fusion of a bioactive peptide therewith.

Figs. 3 to 5 show bispecific (multispecific) antibodies different in antigen-binding region of an antibody and these bispecific (multispecific) antigens can also be given as examples of the protein having at least an antigen-binding region of an antibody in the present invention. This means that the protein having at least an antigen-binding region of an antibody may be an antibody-like molecule.

Figs. 3 to 5 include proteins different in antigen-binding region of an antibody, but the proteins having at least an antigen-binding region of an antibody according to the present invention may be a protein shown in Figs. 3 to 5 and having the same antigen-binding site of an antibody.

### [Site present in a folded portion of a secondary structure in a structural region having an antigen-binding activity and exposed on the surface of a protein]

In the artificial protein of the present invention, the bioactive peptide is fused with a protein having at least an antigen-binding region of an antibody, more specifically, it is fused with a site of the protein present in a folded portion of a secondary structure in a structural region having an antigen-binding activity and exposed on the surface of the protein.

The site of an amino acid sequence, which corresponds to the folded portion of a secondary structure in a structural region having an antigen-binding activity and with which a bioactive peptide is fused, may be present between amino acids adjacent to each other. The site of an amino acid sequence, which corresponds to the folded portion of a secondary structure in a structural region having an antigen-binding activity and with which a bioactive peptide is fused, may be present between amino acids of the amino acid sequence separated from each other. When the bioactive peptide is fused between amino acids separated from each other, the artificial protein may lose one or more amino acids present between the amino acids of the amino acid sequence, which corresponds to the folded portion of a secondary structure in a structural region having an antigen-binding activity, selected for bonding with the bioactive peptide.

The site present in a folded portion of a secondary structure in a structural region having an antigen-binding activity and exposed on the surface of the protein is preferably a site present in a loop region or an elbow region of an antibody Fab fragment (Fab).

A site with which the bioactive peptide is fused may arbitrarily be selected from the loop region or elbow region of the antibody Fab fragment.

The site present in a folded portion of a secondary structure in a structural region having an antigen-binding activity and exposed on the surface of the protein may be present in a Fv region or outside the Fv region. When the site is outside the Fv region, it is preferably present in a structural region not capable of existing stably independent of Fv, for example, a CH1 domain of the H chain or a CL domain of the L chain of the antibody Fab fragment (Fab) present adjacent to the Fv region. In this case, it is preferred that an independent domain (for example, an Fc region of an antibody or an artificially fused Fn3 module) simply connected to Fv or Fab is not used as a site to be fused with the bioactive peptide.

In the present invention, the site present in a folded portion of a secondary structure in a structural region having an antigen-binding activity and exposed on the surface of a protein is preferably a portion called "loop region" of an antibody or a derivative thereof.

Examples of the loop region of an antibody or a derivative thereof include, in H chain, GA loop (VH-CH1), AB loop (CH1), CD loop (CH1), EF loop (CH1), AB loop(VH), C"D loop (VH), EF loop (VH), DE loop (CH1), FG loop (CH1), and CC' loop (VH) and include, in L chain, GA loop (VL-CL), AB loop (CL), CD loop (CL), EF loop (CL), AB loop (VL), C"D loop (VL), EF loop (VL), DE loop (CL), FG loop (CL), and CC' loop (VL).

It is to be noted that a preferable loop region can be selected as needed, depending on the protein having at least an antigen-binding region of an antibody. When the protein having at least an antigen-binding region of an antibody is an antibody or Fab, the loop region may be selected from the above-described ones. This means that a loop region present in the structure which the protein having at least an antigen-binding region of an antibody has may be selected.

In the present invention, as shown in Figs. 48 and 49, when amino acid residue numbers are described using Chothia numbering, loop regions in H chain are preferably:;
GA loop (VH-CH1): positions 112-119,
AB loop (CH1): positions 125-134,
CD loop (CH1): positions 155-162,
EF loop (CH1): positions 186-194,
AB loop (VH): positions 13-17,
C"D loop (VH): positions 61-66,
EF loop ver1 (VH): positions 82b-87,
DE loop (CH1): positions 172-174,
FG loop (CH1): positions 201-203, and
CC' loop (VH): positions 40-44, and those in L chain are preferably:
   GA loop (VL-CL): positions 105-113,
   AB loop (CL): positions 119-128,
   CD loop (CL): positions 151-158,
   EF loop (CL): positions 182-190,
   AB loop (VL): positions 13-18,
   C"D loop (VL): positions 57-61,
   EF loop (VL): positions 76-83,
   DE loop (CL): positions 165-174,
   FG loop (CL): positions 198-203, and
   CC' loop (VL): positions 39-44.

In the present invention, the folded portion of a secondary structure in a structural region having an antigen-binding activity has preferably a loop structure which can be identified by the Chothia number. The site present in the folded portion of a secondary structure in a structural region having an antigen-binding activity and exposed on the surface of the protein may be either the whole loop structure or a portion of the loop structure.

This means that the site present in the folded portion of a secondary structure in a structural region having an antigen-binding activity and exposed on the surface of the protein may be preferably a site in a loop structure which can be identified by the Chothia number.

A description will be made with the GA loop (VH-CH1) as an example, the GA loop (VH-CH1) is defined as an amino acid at positions 112-119 according to Chothia number. Although the site present in a folded portion of a secondary structure in a structural region having an antigen-binding activity and exposed on the surface of the protein is not particularly limited by using Chothia numbering, it may be selected as a site from an arbitrary one of positions 112, 113, 114, 115, 116, 117, and 118 to an arbitrary one of positions 119, 118, 117, 116, 115, 114, and 113 such as positions 112-113, positions 113-114, positions 114-115, positions 115-116, positions 116-117, positions 117-118, positions 118-119, positions 112-114, positions 113-115, positions 114-116, positions 115-117, positions 116-118, positions 117-119, positions 112-115, positions 113-116, positions 114-117, positions 115-118, positions 116-119, positions 112-116, positions 113-117, positions 114-118, positions 115-119, positions 112-117, positions 113-118, positions 114-119, positions 112-118, and positions 113-119.

As an arbitrary position thus selected, the protein may be bonded to the N terminal side of a bioactive peptide at a position where the amino acid number based on Chothia numbering is small or the protein may be bonded to the C terminal side of the bioactive peptide at a position where the amino acid number based on Chothia numbering is large.

The GA loop (VH-CH1) is defined as an amino acid at positions 112 to 119 according to Chothia number. But if it is a site exposed on the surface of the protein, it may be selected from positions on the N terminal side from position 112 or similarly, it may be selected from positions on the C terminal side from position 119.

In the present invention, in each of the artificial proteins, a loop structure corresponding to the amino acid number defined by Chothia numbering may be selected.

The insertion site of the bioactive peptide in the loop region is preferably in a B1, B2, B2-2, B3, M1, M2, M2-2, M3, M3-2, M4, M5, M5-2, or M6 site in L chain shown in Fig. 7 or Fig. 8; or in a B1, B2, B2_2, B3, M1, M2, M2-2, M3, M3-2, M4, M5, or M6 site in H chain shown in Fig. 14 or Fig. 15.

According to Fig. 6, the insertion site is as follows (N1_mP6-9_M6_{L}).

...YQQKP⁴⁰-(GWRPYIERWTGRLIVGG)-G⁴¹KAPK... According to Fig. 13, the insertion site is as follows (N1_mP6-9_M6_{H}).

...VROA^{p41}-(GWRPYIERWTGRLIVGG)-G⁴²KGLE... The insertion site of the bioactive peptide in the elbow region is preferably in the elbow site in L chain shown in Fig. 7 or in the elbow site in H chain shown in Fig. 14.

In Fig. 7, Fig. 8, Fig. 14, and Fig. 15, each insertion site is shown with reference to the structure of the antibody (1HZH). In the case of another antibody, a site of the another antibody corresponding to the site in the antibody (1HZH) can be used as an insertion site.

Examples of the insertion site of the bioactive peptide include those shown in Fig. 6 and Fig. 13.

In the present invention, it is preferred that the insertion sites in the H chain according to the Chothia number is positions 113-114 as an E site in the GA loop (VH-CH1), positions 132-133 as a B1 site in the AB loop (CH1), positions 156-157 as a B2 site and positions 160-161 as a B2-2 site, each in the CD loop (CH1), positions 190-191 as a B3 site in the EF loop (CH1), positions 14-15 as an M1 site in the AB loop (VH), positions 61-62 as an M2 site and positions 65-66 as an M2-2 site, each in the C"D loop (VH), positions 82a-82b as an M3 site and positions 83-84 as an M3-2 site, each in the EF loop (VH), positions 172-173 as an M4 site in the DE loop (CH1), positions 203-204 as an M5 site in the FG loop (CH1), and positions 41-42 as an M6 site in the CC' loop (VH). It is preferred that the insertion sites in the L chain are positions 108-109 as E site in the GA loop (VL-CL), positions 127-128 as a B1 site in the AB loop (CL), positions 151-152 as a B2 site and positions 156-157 as B2-2 site, each in the CD loop (CL), positions 188-189 as a B3 site in the EF loop (CL), from positions 15-16 as M1 site in the AB loop (VL), positions 56-57 as an M2 site and positions 60-61 as a M2 site, each in the C"D loop (VL), positions 76-77 as an M3 site and positions 80-81 as a M3-2 site, each in the EF loop (VL), positions 169-170 as a M4 site in the DE loop (CL), positions 202-203 as an M5 site and positions 199-200 as an M502 site, each in the FG loop (CL), and positions 40-41 as an M6 site in the CC' loop (VL).

It is to be noted that in Fig. 6 and Fig. 13, the H chain and the L chain are shown by attaching subscripts H and L, respectively.

In the present invention, when the protein having at least an antigen-binding region of an antibody is an antibody, Fab, or the like, a B1 site, a B2 site, or a B3 site is preferred in the H chain, while a B1 site, a B2 site, a B2-2 site, or a B3 site is preferred in the L chain, each site becoming a so-called bottom site.

In the present invention, since a bioactive peptide can be fused with the vicinity of the antigen-binding region, an insertion site other than the so-called bottom site can be preferably used.

In the H chain, a loop region to which the B1 site, the B2 site, or the B3 site belongs is preferred, while in the L chain, a loop region to which the B1 site, the B2 site, the B2-2 site, or the B3 site is preferred.

### [Production of an artificial protein having a bioactive peptide fused with a site, of a protein having at least an antigen-binding region of an antibody, present in a folded portion of a secondary structure in a structural region having an antigen-binding activity and exposed on the surface of the protein]

In the present invention, no particular limitation is imposed on a method of producing an artificial protein having a bioactive peptide fused with a site, of a protein having at least an antigen-binding region of an antibody, present in a folded portion of a secondary structure in a structural region having an antigen-binding activity and exposed on the surface of the protein and the artificial protein of the present invention can be produced using the method described in Example. For the production, a conventionally known gene engineering method or a modification thereof can be used.

A method of producing an artificial protein having a bioactive peptide fused, in a protein having at least an antigen-binding region of an antibody, with a site present in a folded portion of a secondary structure in a structural region having an antigen-binding activity and exposed on the surface of the protein according to the present invention includes:
selecting all or some of amino acid sequences to be fused onto a protein from the amino acid sequences of a bioactive peptide and selecting base sequences corresponding to the amino acid sequences;
selecting a site, in the protein having at least an antigen-binding region of an antibody, present in a folded portion of a secondary structure in a structural region having an antigen-binding activity and exposed on the surface of the protein;
selecting base sequences corresponding to two amino acid residues constituting the site with which the bioactive peptide is fused, deleting a base present between the base sequences thus selected if necessary, inserting and incorporating the base sequences corresponding to the selected amino acid sequences derived from the bioactive peptide, and thus preparing a nucleic acid having the resulting base sequence; and
translating the nucleic acid.

The present invention also provides a method of presenting a bioactive peptide on a protein having at least an antigen-binding region of an antibody, that is, a method of presenting a bioactive peptide on a protein by fusing the bioactive peptide with a site, in the protein, present in a folded portion of a secondary structure in a structural region having an antigen-binding activity and exposed on the surface of the protein.

Since in the artificial protein of the present invention having a bioactive peptide fused with a site, of a protein having at least an antigen-binding region of an antibody, present in a folded portion of a secondary structure in a structural region having an antigen-binding activity and exposed on the surface of the protein, a "bioactive peptide" is fused with a "site, in a protein having at least an antigen-binding region of an antibody, present in a folded portion of a secondary structure in a structural region having an antigen-binding activity and exposed on the surface of the protein", a method of presenting a bioactive peptide on a protein can be provided similarly as described in the artificial protein of the present invention.

It is also possible to understand that in the present invention, the "method of presenting a bioactive peptide on a protein having at least an antigen-binding region of an antibody" is "a method of fusing a bioactive peptide with a protein having at least an antigen-binding region of an antibody to give multispecificity to the protein" by fusing the bioactive peptide with a site of the protein present in a folded portion of a secondary structure in a structural region having an antigen-binding activity and exposed on a surface of the protein.

In other words, the protein having at least an antigen-binding region of an antibody has preferably multispecificity by retaining its antigen binding activity derived from the antibody and being imparted with a function derived from a fused bioactive peptide.

### Examples

The present invention will hereinafter be described specifically by Examples, but it should be noted that the resent invention is not limited by the following Examples.

### Fusion of Fab region which is an antigen-binding site with bioactive peptide

A peptide fusion protein was prepared as described below by inserting a bioactive peptide into a Fab region, an antigen-binding site, of an IgG full-length protein (which will hereinafter be called "IgG").

Fig. 1 shows the structure of an antibody (1HZH) as an example. Fig. 7, Fig. 8, Fig. 14, and Fig. 15 show an insertion site of the bioactive peptide with reference to the structure of the antibody shown in Fig. 1.

### [Example 1]

### 1. Preparation of peptide fusion protein

A DNA encoding the Fab region of the H chain of a human IgG monoclonal antibody (N1 antibody, clone name: YW64.3) against neuropilin 1 and a DNA encoding a Hisx6 tag were incorporated in an expression vector pcDNA3.1 (product of Thermo Fisher Scientific) (antibody H-chain Fab region expression vector).

The amino acid sequence (SEQ ID NO: 2) of the N1 antibody H-chain Fab region produced by the resulting vector is shown in Fig. 48. The amino acid sequence (SEQ ID NO: 1) of the N1 antibody H chain is shown in Fig. 47.

A DNA encoding the LK chain full-length region of the N1 antibody was incorporated in the expression vector pcDNA 3.1 (antibody L_{K} chain expression vector). It was incorporated without a tag. The amino acid sequence (SEQ ID NO: 3) of the N1 antibody L_{K} chain produced by the resulting vector is shown in Fig. 49.

As the bioactive peptide, mP6-9 having a binding activity to human plexin B1 was selected. The mP6-9 is a cyclic peptide having an amino acid sequence (SEQ ID NO: 4) of D-Trp Arg Pro Tyr Ile Glu Arg Trp Thr Gly Arg Leu Ile Val and Cys and its cyclic structure is composed of chloroacetylated D-Trp and Cys. The amino acid sequence (SEQ ID NO: 5) of mP6-9 inserted in each site of the Fab region is Trp Arg Pro Tyr Ile Glu Arg Trp Thr Gly Arg Leu Ile and Val.

As shown in Fig. 6 and Fig. 13, Gly was used as a linker for the N terminal and the C terminal of the inserted amino acid sequence (SEQ ID NOS: 6 to 32).

An antibody H chain-peptide fusion vector was prepared by selecting, based on the antibody H-chain Fab region expression vector, an elbow (E) site of the region, or as a loop region, B1, B2, B2-2, B3, M1, M2, M2-2, M3, M3-2, M4, M5, or M6 site, and inserting a DNA sequence corresponding to the mP6-9 sequence into the site. For example, insertion into the elbow (E) site in the H chain was achieved by inserting a DNA sequence (SEQ ID NO: 20) encoding an amino acid sequence including the mP6-9 sequence in the following parentheses between serine (S) at position 113 and alanine (A) at position 114 (the amino acid residue number according to Chothia numbering) as shown below. ...VTVSS¹¹³-(GWRPYIERWTGRLIVG)-A¹¹⁴STKG...

Similarly, the insertion position into each site in the H chain is shown in Fig. 13.

The Fab proteins including the peptide fusion type antibody H chain were formed by co-expression of the antibody H chain-peptide fusion vector and an unfused antibody L_{K} chain expression vector and they were named in the following order: N1_Fab_(name of peptide)_(name of site) (Fig. 16 to Fig. 18), for example, N1_Fab_mP6-9_M1_{H} .

An antibody L chain-peptide fusion vector was prepared by selecting, based on the antibody L_{K} chain expression vector, an elbow (E) site in the region or, as a loop region, B1, B2, B2_2, B3, M1, M2, M2-2, M3, M3-2, M4, M5, M5-2, or M6 site, and inserting a DNA sequence corresponding to the mP6-9 sequence into the site. For example, insertion into the elbow (E) site in the L chain was achieved by inserting a DNA sequence (SEQ ID NO: 6) encoding an amino acid sequence including the mP6-9 sequence in the following parentheses between arginine (R) at position 108 and threonine (T) at position 109 (the amino acid residue number according to Chothia numbering) as shown below. ...vEIKR¹⁰⁸-(GWRPYIERWTGRIVG)-T¹⁰⁹V-AAP...

Similarly, the insertion position into each site in the L chain is shown in Fig. 6.

The Fab proteins including the peptide fusion type antibody L chain were formed by co-expression of the antibody L chain-peptide fusion vector and an unfused antibody H-chain Fab region expression vector and they were named in the following order: N1_Fab_(name of peptide)_(name of site) (Fig. 9 to Fig. 12), for example, N1_Fab_mP6-9_M1_{L}.

The peptide fusion type antibody H chain and the peptide fusion type L chain are shown, respectively, by subscripts H and L in the site name.

A peptide fusion type Fab protein (Fabbody) including the peptide fusion type antibody H chain or the peptide fusion type antibody L chain were prepared by co-expressing the antibody H chain-peptide fusion vector and the unfused antibody L_{K} chain expression vector or the antibody L chain-peptide fusion vector and the unfused antibody H-chain Fab region expression vector by using Expi293F cells (product of Thermo Fischer Scientific) and thereby secreting the coexpression product in the culture supernatant. The culture and transfection of the cells was performed following the standard protocol of Thermo Fischer Scientific.

A sample was obtained by adding 40 µL of Ni-NTA Sepharose (product of Qiagen) to 0.25 mL of the collected culture supernatant, mixing the resulting mixture by rotation for 2 hours, precipitating the Sepharose by centrifugal separation to remove the supernatant, washing the Sepharose twice with 1 mL of Tris-buffered saline (TBS, 20 mM Tris-HCI, 150 mM NaCl, pH 7.5), adding 12.5 µL of 500 mM Imidazole having a pH of 8.0 to TBS for elution, adding 12.5 µL of an SDS sample buffer further, and heating the mixture at 95°C for 2 minutes. The sample (5 µL) thus obtained by elution was subjected to electrophoresis under non-reducing conditions and stained with Coomassie brilliant blue. The results are shown in Fig. 9 and Fig. 16.

### 2. Binding of peptide fusion protein to target molecule

A pulldown type binding test was performed to study whether or not the N1_Fab (mP6-9 peptide fusion type N1_Fab) obtained by fusing an mP6-9 peptide with various sites retained both binding ability to neuropilin 1 which the N1 antibody had before fusion and binding ability to human plexin B1 which the mP6-9 peptide had before fusion. According to the method described in Protein Exp. Purification, 2014; 95: 240-247, a construct encoding a fusion protein having a PA tag (product of Wako Pure Chemical Industries) added to the C terminal of the extracellular region of human plexin B1 and a construct encoding a fusion protein having a PA tag added to the C terminal of the extracellular region of mouse neuropilin 1 were prepared and they were incorporated in an expression vector pcDNA3.1 (product of Thermo Fisher Scientific). Transient expression was caused in Expi293F cells by using the resulting vectors according to the above-described method and culture supernatants containing plexin B1-PA and neuropilin 1-PA, each a soluble receptor fragment, were prepared.

As described above, after capturing the mP6-9 peptide fusion type N1_Fab on Ni-NTA Sepharose, the culture supernatant containing plexin B1-PA or neuropilin 1-PA, each prepared separately by the above-described method, was caused to react. After washing the Sepharose and eluting the bound protein as described above, electrophoresis was performed under non-reducing conditions, followed by staining with Coomassie brilliant blue. The results are shown in Fig. 10 and Fig. 11 and Fig. 17 and Fig. 18.

The data thus obtained show the test results suggesting that the mP6-9 peptide fusion type N1_Fab has binding ability to both neuropilin 1 and human plexin B.

A pulldown type binding test was performed to study whether or not the mP6-9 peptide fusion type N1_Fab had simultaneous binding ability to neuropilin 1 and human plexin B1.

A construct encoding a fusion protein having, added at the C terminal of the extracellular region of mouse neuropilin 1, DYKDDDDKtag (SEQ ID NO: 33) was formed and the resulting construct was incorporated in an expression vector pcDNA3.1 (product of Thermo Fisher Scientific). Transient expression was performed in Expi293F cells by using the vector according to the above-described method and a culture supernatant containing neuropilin 1-DYKDDDDK, a soluble receptor fragment, was prepared. The culture supernatant (0.25 mL) containing neuropilin 1-DYKDDDDK was captured on 40 µL of Anti DYKDDDDK tag Antibody Beads Sepharose (product of Wako Pure Chemical Industries). After the Sepharose was precipitated by centrifugal separation to remove the supernatant and was washed once with 1 mL of Tris-buffered saline (TBS, 20 mM Tris-HCI, 150 mM NaCl, pH 7.5), 0.25 mL of a culture supernatant containing a mP6-9 peptide fusion Fab was added. They were mixed by rotation for one hour and reacted. The Sepharose was precipitated again by performing centrifugal separation and washed once with 1 mL of TBS. Then, 0.25 mL of a culture supernatant containing human plexin B1-PA was added and they were reacted by mixing by rotation for one hour. The Sepharose was then washed twice with 1 mL of TBS. TBS (12.5 µL) and 12.5 µL of an SDS sample buffer were added for elution, followed by heating at 95°C for 2 minutes to obtain a sample. The sample (10 µL) thus obtained by elution was subjected to electrophoresis under non-reducing conditions and stained with Coomassie brilliant blue. The results are shown in Fig. 12 and Fig. 19.

The data thus obtained show the test results suggesting that the mP6-9 peptide fusion type N1_Fab has simultaneous binding ability to neuropilin 1 and human plexin B.

### 3. Simultaneous binding ability of peptide fusion protein to two target molecules

### 3-1. Principle of sandwich type intermolecular crosslinking test and preparation of target antigen

A sandwich ELISA type assay system was constructed to show that the mP6-9 peptide fusion type N1_Fab could simultaneously bind to two kinds of target molecules. The principle is shown in Fig. 20A. First, according to the method described in Protein Exp. Purification, 2014; 95: 240-247, a construct encoding a fusion protein having a PA tag (product of Wako Pure Chemical Industries) added to the C terminal of the extracellular region of human plexin B1 as the second target molecule was formed and the resulting construct was incorporated in an expression vector pcDNA 3.1 (product of Thermo Fisher Scientific). Transient expression was caused in Expi293F cells by using the resulting vector according to a method similar to that described above in "2." to prepare a culture supernatant containing plexin B1-PA, a soluble receptor fragment, followed by purification with anti-PA tag antibody beads (product of Wako Pure Chemical Industries). On the side of the first antigen, pAPtag-5 (product of GenHunter), an AP fusion expression vector, was used to fuse the sequence of the extracellular region thereof with the N terminal (neuropilin 1) of AP. By transient expression in Expi293F cells according to a method similar to that described above in "2", a culture supernatant containing Nrp1 ec-AP, a soluble receptor-AP fusion product was prepared.

### 3-2. Sandwich type intermolecular crosslinking test

The crosslink between the two target molecules by the mP6-9 peptide fusion type N1_Fab was evaluated in accordance with the following protocol based on the principle shown in Fig. 20A.
(1) 50 µL of a purified second target antigen solution diluted to 10 µg/mL was added to a 96 well plate and the plate was allowed to stand at room temperature for 3 hours.
(2) After the purified second target antigen solution was removed, 200 µL/well of Blocking One (product of Nacalai Tesque) diluted to 10-fold with TBS (TBS; 20 mM Tris-HCI, 150 mM NaCl, pH 7.5) was added and the plate was allowed to stand overnight at 4°C.
(3) The plate was washed once with 200 µL/well of TBS.
(3) 50 µL of an expression supernatant of the mP6-9 peptide fusion type N1_Fab was added and the plate was allowed to stand at room temperature for 2.5 hours.
(4) The plate was washed three times with 200 µL/well of TBS.
(5) 50 µL of a supernatant expressing an AP fusion first target antigen was added and the plate was allowed to stand at room temperature for 2 hours.
(6) The plate was washed four times with 200 µL/well of TBS and lastly, 100 µL/well of TBS was added.
(7) After 100 µL/well of a chromogenic substrate (Phosphatase substrate, product of Sigma Aldrich) was added further and the plate was allowed to stand at 37°C for 30 minutes, absorbance at 405 nm of the solution in each well was measured. The results are shown in Fig. 20B.

The mP6-9 peptide fusion type N1_Fab was captured on the plate having the second target antigen immobilized thereon and was bound to the first target antigen-AP fusion protein added later to cause the substrate to develop a color. On the other hand, the N1_Fab having no peptide inserted therein did not give a positive signal. This shows that Fabbody, a peptide fusion type Fab protein, is capable of, not individually, but simultaneously bonding to the first and second targets.

### [Example 2]

### 1. Preparation of peptide fusion protein

A peptide fusion type IgG protein containing a peptide fusion type antibody H chain or a peptide fusion type antibody L chain was prepared by co-expressing an antibody H chain-peptide fusion vector and an unfused L_{K} chain expression vector or an antibody L chain-peptide fusion vector and an unfused antibody H-chain IgG region expression vector by using Expi293F cells (product of Thermo Fischer Scientific) and thereby secreting the co-expression product in the culture supernatant.

A sample was obtained by adding 40 µL of Protein A Sepharose (product of GE Health Care) to 0.25 mL of the collected culture supernatant, mixing the resulting mixture by rotation for 1 hour, precipitating the Sepharose by centrifugal separation to remove the supernatant, washing the Sepharose twice with 1 mL of Tris-buffered saline (TBS, 20 mM Tris-HCI, 150 mM NaCl, pH 7.5), adding 12.5 µL of TBS and 12.5 µL of an SDS sample buffer to cause elution, and heating at 95°C for 2 minutes. The sample (5 µL) thus obtained by elution was subjected to electrophoresis under reducing conditions and stained with Coomassie brilliant blue. The results are shown in Fig. 21.

### 2. Binding of peptide fusion protein to target molecule

A pulldown type binding test was performed to study whether or not N1_IgGs (mP6-9 peptide fusion type N1_IgGs) having an mP6-9 peptide fused at various sites thereof retained both binding ability to neuropilin 1 which the N1 antibody had before fusion and binding ability to human plexin B1 which the mP6-9 peptide had before fusion.

After the mP6-9 peptide fusion type N1_IgG was captured on Protein A Sepharose, a culture supernatant containing plexin B1-PA or neuropilin 1-PA, which had been prepared separately by a method similar to that of Example 1, was caused to react with it. After washing the Sepharose and eluting the bound protein as described above, electrophoresis was performed under reducing conditions, followed by staining with Coomassie brilliant blue. The results are shown in Fig. 22 and Fig. 23.

The data thus obtained show the test results suggesting that the mP6-9 peptide fusion IgG has binding ability to both neuropilin 1 and human plexin B1.

### [Example 3]

The present example was performed in a manner similar to that of Example 1 except that as the bioactive peptide, mP6-9 having a binding activity to human plexin B1 was replaced by aMD4 having a binding activity to a human Met receptor (Met).

The aMD4 is a cyclic peptide having the following amino acid sequence (SEQ ID NO: 34): D-Tyr Arg Gln Phe Asn Arg Arg Thr His Glu Val Trp Asn Leu Asp Cys in which chloroacetylated D-Tyr and Cys form a cyclic structure. The aMD4 inserted in each site of the Fab region has the following amino acid sequence (SEQ ID NO: 35): Tyr Arg Gln Phe Asn Arg Arg Thr His Glu Val Trp Asn Leu Asp.

As shown in Fig. 24 and Fig. 28, the amino acid sequence (SEQ ID NOS: 36 to 51) thus inserted has, at the N terminal and C terminal thereof, Gly serving as a linker.

The results are shown in Fig. 25 to Fig. 27 and Fig. 29 to Fig. 32.

### [Example 4]

### 1. Preparation of peptide fusion protein

The present example was performed as in Example 1 except that as the Fab protein, the N1 antibody was replaced by the Fab of an anti-CD3 antibody (OKT3 antibody).

Antibody H chain-peptide fusion vectors were prepared by selecting, based on an OKT3 antibody H-chain Fab region expression vector, B1, B2, B2-2, and B3 sites as a loop region and inserting therein a DNA sequence corresponding to the aMD4 sequence or aMD5 sequence used in Example 3. Antibody L chain-peptide fusion vectors were prepared by selecting B1, B2, B2_2, and B3 sites as a loop region based on an OKT3 antibody L_{K} chain expression vector and inserting therein a DNA sequence corresponding to the aMD4 sequence or aMD5 sequence. The insertion site of the bioactive peptide in the H chain or L chain is a site corresponding thereto in the Fab of the OKT3 antibody according to the insertion site shown in Fig. 24 and Fig. 28 and the Chothia numbering based on amino acid residue number.

The aMD4 is similar to that of Example 3.

The aMD5 is a cyclic peptide having the following amino acid sequence (SEQ ID NO: 52) of D-Tyr Trp Tyr Tyr Ala Trp Asp Gln Thr Tyr Lys Ala Phe Pro Cys in which chloroacetylated D-Tyr and Cys form a cyclic structure. The amino acid sequence (SEQ ID NO: 53) of the aMD5 inserted in each site of the Fab region is: Tyr Trp Tyr Tyr Ala Trp Asp Gln Thr Tyr Lys Ala Phe Pro. The amino acid sequence thus inserted has, at the N terminal and the C terminal thereof, Gly serving as a linker.

Fig. 33 shows the results obtained by, in a manner similar to that of Example 1, carrying out co-expression by using Expi293F cells (product of Thermo Fisher Scientific), capturing the culture supernatant on Ni-NTA Sepharose, and performing electrophoresis after washing and elution.

### 2. Binding of peptide fusion protein to target molecule

A pulldown type binding test was performed to study whether or not the peptide fusion protein retained binding ability to a human Met receptor (Met) which the aMD4 or MD5 peptide had before fusion. A sample was prepared as follows: 1.0 mL of a culture supernatant containing Met-PA, a soluble receptor fragment, transiently expressed in Expi293F cells was captured on anti-PA tag antibody beads (product of Wako Pure Chemical Industries). After the Sepharose was precipitated by centrifugal separation to remove the supernatant and was washed once with 1 mL of Tris-buffered saline (TBS, 20 mM Tris-HCI, 150 mM NaCl, pH 7.5) , 0.8 mL of the supernatant containing the peptide fusion Fab prepared above was added and they were reacted by mixing for one hour by rotation. The Sepharose was precipitated again by centrifugal separation and washed twice with 1 mL of TBS. Then, 12.5 µL of TBS and 12.5 µL of a SDS sample buffer were added to cause elution and the elution product was heated at 95°C for 2 minutes. The sample (12 µL) thus obtained by elution was subjected to electrophoresis under non-reducing conditions, followed by staining with Coomassie brilliant blue. The results are shown in Fig. 34.

### 3. Binding of peptide fusion protein to target molecule on cell surface

A binding test by flow cytometry was performed to study whether or not the aMD4 peptide fusion type OKT3_Fabs having, inserted in various positions thereof, the aMD4 peptide retained binding ability to a human Met receptor (Met). Respective supernatants of OKT3_Fab and six kinds of aMD4 peptide fusion type OKT3_Fabs were reacted with CD3-expressing human T lymphocyte leukemia-derived Jurkat cells or human Met receptor stable expression CHO cells and thus bound Fabs were stained with an AlexaFluor488-labeled goat anti-human IgG secondary antibody (product of Thermo Fisher Scientific) and analyzed using an EC800 type flow cytometer (product of SONY). The results are shown in Fig. 35.

In the upper column, whether or not plain-Fab (OKT3-Fab) and aMD4 peptide fusion type OKT3_Fab bind to Jurkat cells which express CD3 on the cell surface is confirmed. Binding can be confirmed from a shift of the light blue region to the right side relative to the gray region of no-Fab. This suggests that even the aMD4 peptide fusion type OKT3_Fab does not lose its essential binding ability to a CD3 antigen. In the lower column, the binding ability to Met added by insertion of the aMD4 peptide is studied. The shift can be observed in the aMD4 peptide fusion type OKT3_Fab so that binding to Met-CHO (expression of Met on cell surface) can be confirmed. On the other hand, no shift can be observed in the plain-Fab (OTK3-Fab). It can be confirmed from such a finding that the aMD4 peptide fusion type OKT3_Fab has new Met-specific binding ability due to the aMD4 peptide fused therewith.

### [Example 5]

The present example was performed as in Example 4 except that as the Fab protein, the Fab of an anti-CD3 antibody (UCHT1 antibody) was used instead of the N1 antibody. The results are shown in Fig. 36 to Fig. 38.

It can be confirmed that the aMD4 peptide fusion type UCHT1_Fab has also new Met-specific binding ability due to the aMD4 peptide fused therewith.

### [Example 6]

### 1. Preparation of peptide fusion protein

The present example was performed as in Example 4 except that as the Fab protein, the Fab of an anti-CD16 antibody (3G8 antibody) was used instead of the N1 antibody. The results are shown in Fig. 39.

### 2. Binding of peptide fusion protein to target molecule

A pulldown type binding test was performed to study whether or not the resulting peptide fusion protein retained the binding ability to CD16 which the 3G8 antibody had before fusion. A sample was obtained as follows: 0.5 mL of a culture supernatant containing CD16-PA, a soluble receptor fragment, transiently expressed in Expi293F cells was captured on anti-PA tag antibody beads (product of Wako Pure Chemical Industries). By centrifugal separation, the Sepharose was precipitated to remove the supernatant. After the Sepharose was washed once with 1 mL of Tris-buffered saline (TBS, 20 mM Tris-HCI, 150 mM NaCl, pH7.5), 0.25 mL of a culture supernatant containing the peptide fusion Fab produced above was added and they were reacted by mixing for one hour by rotation. The Sepharose was precipitated again by centrifugal separation and washed twice with 1 mL of TBS. Then, 12.5 µL of TBS and 12.5 µL of a SDS sample buffer were added to cause elution and the elusion product was heated at 95°C for 2 minutes. The sample (20 µL) thus obtained by elution was subjected to electrophoresis under non-reducing conditions, followed by staining with Coomassie brilliant blue. The results are shown in Fig. 40.

A pulldown type binding test was performed to study whether or not the peptide fusion protein retained the binding ability to a human Met receptor (Met) which the aMD4 or MD5 peptide had before fusion. A sample was obtained as follows: 0.5 mL of a culture supernatant containing Met-PA, a soluble receptor fragment, transiently expressed in Expi293F cells was captured on anti-PA tag antibody beads (product of Wako Pure Chemical Industries). By centrifugal separation, the Sepharose was precipitated to remove the supernatant. After the Sepharose was washed once with 1 mL of Tris-buffered saline (TBS, 20 mM Tris-HCI, 150 mM NaCl, pH 7.5), 0.25 mL of a culture supernatant containing the peptide fusion Fab produced above was added and they were reacted by mixing for one hour by rotation. The Sepharose was precipitated again by centrifugal separation and was washed twice with 1 mL of TBS. Then, 12.5 µL of TBS and 12.5 µL of a SDS sample buffer were added to cause elution and the elution product was heated at 95°C for 2 minutes. The sample (20 µL) thus obtained by elution was subjected to electrophoresis under non-reducing conditions, followed by staining with Coomassie brilliant blue. The results are shown in Fig. 41.

### [Example 7]

### 1. Preparation of peptide fusion protein (trispecific)

An antibody H-chain-mP6-9 peptide fusion vector was prepared by selecting, based on the antibody H-chain Fab region expression vector described in Example 1, the elbow (E) site of the Fab region thereof or as the loop region, a B1, B2_2, or M4 site and incorporating therein a DNA sequence corresponding to the mP6-9 sequence.

An antibody L-chain-mP6-9 peptide fusion vector was prepared by selecting, based on the antibody L_{K} chain expression vector described in Example 1, the elbow (E) site of the Fab region thereof or as the loop region, a B1, M1, or M2-2 site and incorporating therein a DNA sequence corresponding to the mP6-9 sequence.

An antibody H chain-aMD4 peptide fusion vector was prepared by selecting, based on the antibody H-chain Fab region expression vector described in Example 1 , the elbow (E) site of the Fab region thereof, or as the loop region, a B1, B2, M4, or M5 site and incorporating therein a DNA sequence corresponding to the aMD4 sequence.

An antibody L chain-aMD4 peptide fusion vector was prepared by selecting, based on the antibody L_{K} chain expression vector described in Example 1, the elbow (E) site of the Fab region thereof or as the loop region, a B2-2, M2, or M2-2 site and incorporating therein a DNA sequence corresponding to the aMD4 sequence.

The insertion site of the bioactive peptide in the H chain or L chain is an insertion site shown in Fig. 6 or Fig. 13.

A trispecific peptide fusion type Fab protein was prepared by coexpressing the antibody H chain-mP6-9 peptide fusion vector and the antibody L chain-aMD4 peptide fusion vector or the antibody H chain-aMD4 peptide fusion vector and the antibody L chain-mP6-9 peptide fusion vector in Expi293F cells (product of Thermo Fisher Scientific) and thereby secreting them in the culture supernatant. Cell culture or transfection was performed following the standard protocol of Thermo Fisher Scientific.

A sample was obtained by adding 40 µL of Ni-NTA Sepharose (product of Qiagen) to 0.25 mL of the collected culture supernatant, mixing the resulting mixture by rotation for 1 hour, precipitating the Sepharose by centrifugal separation to remove the supernatant, washing the Sepharose twice with 1 mL of Tris-buffered saline (TBS, 20 mM Tris-HCI, 150 mM NaCl, pH 7.5), adding 12.5 µm of 500 mM imidazole having a pH of 8.0 to TBS to cause elution, adding 12.5 µL of an SDS sample buffer; and heating the resulting mixture at 95°C for 2 minutes. The sample (5 µL) thus obtained by elution was subjected to electrophoresis under non-reducing conditions and stained with Coomassie brilliant blue. The results are shown in Fig. 42.

### 2. Binding of peptide fusion protein (trispecific) to target molecule

A pulldown type binding test was performed to study whether or not the trispecific peptide fusion type Fab protein retained each of binding ability to neuropilin 1, binding ability to human plexin B1 which the mP6-9 peptide had before fusion, and binding ability to a human Met receptor (Met) which the aMD4 peptide had before fusion. According to the method described in Protein Exp. Purification, 2014; 95: 240-247, a construct encoding a fusion protein having a PA tag (product of Wako Pure Chemical Industries) added to the C terminal of the extracellular region of human plexin B1, a construct encoding a fusion protein having a PA tag (product of Wako Pure Chemical Industries) added to the C terminal of the extracellular region of a human Met receptor, and a construct encoding a fusion protein having a PA tag added to the C terminal of the extracellular region of mouse neuropilin 1 were formed and they were each incorporated in an expression vector pcDNA3.1 (product of Thermo Fisher Scientific). The resulting vectors were transiently expressed in Expi293F cells by the above-described method and respective culture supernatants containing plexin B1-PA, Met-PA, and neuropilin 1-PA, each a soluble receptor fragment, were prepared.

A sample was obtained as described below: 1.0 mL of the culture supernatant obtained by transient expression in Expi293F cells and containing any of plexin B1-PA, Met-PA, and neuropilin 1-PA, each a soluble receptor fragment, was captured on anti-PA tag antibody beads (product of Wako Pure Chemical Industries). Sepharose was precipitated by centrifugal separation and the supernatant was removed. After washing the Sepharose once with 1 mL of Tris-buffered saline (TBS, 20 mM Tris-HCI, 150 mM NaCl, pH 7.5), 1.0 mL of the culture supernatant containing the peptide fusion Fab produced above was added and they were reacted by mixing for one hour by rotation. Centrifugal separation was performed again to precipitate the Sepharose. The Sepharose was washed twice with 1 mL of TBS and then, 12.5 µL of TBS and 12.5 µL of a SDS sample buffer were added to cause elution, followed by heating at 95°C for 2 minutes. The sample (5 µL) thus obtained by elution was subjected to electrophoresis under non-reducing conditions, followed by staining with Coomassie brilliant blue. The results with neuropilin 1-PA are shown in Fig. 43, the results with plexin B1-PA are shown in Fig. 44, and the results with Met-PA are shown in Fig. 45N.

It can be confirmed from the results of Fig. 43 to Fig. 45 that the trispecific peptide fusion type Fab protein retains each of binding ability to neuropilin 1, binding ability to human plexin B1 which the mP6-9 peptide has before fusion, and binding ability to a human Met receptor (Met) which the aMD4 peptide has before fusion.

### 3. Simultaneous binding ability of peptide fusion protein to two kinds of target molecules

### 3-1. Principle of sandwich type intermolecular crosslinking test and preparation of target antigen

A sandwich ELISA type assay system was constructed to show that the trispecific peptide fusion type Fab could simultaneously bind to two kinds of target molecules. The principle is shown in Fig. 46A. First, according to the method described in Protein Exp. Purification, 2014, 95, 240-247, a construct encoding a fusion protein having a PA tag (product of Wako Pure Chemical Industries) added to the C terminal of the extracellular region of human plexin B1 or human Met receptor serving as the second target molecule was formed and the resulting construct was incorporated in an expression vector pcDNA 3.1 (product of Thermo Fisher Scientific). The resulting vector was transiently expressed in Expi293F cells by a method similar to that of Example 1 to prepare a culture supernatant containing plexin B1-BA or Met-PA, a soluble receptor fragment, followed by purification with anti-PA tag antibody beads (product of Wako Pure Chemical Industries). On the side of the first antigen, pAPtag-5 (product of GenHunter), an AP fusion expression vector, was used to fuse the sequence of the extracellular region thereof with the N terminal (neuropilin 1) of AP. By transient expression in Expi293F cells by a method similar to that of Example 1, culture supernatants each containing Nrp1ec-AP, a soluble receptor-AP fusion product, were prepared.

### 3-2. Sandwich type intermolecular crosslinkinq test

The crosslink between the target molecules by the trispecific peptide fusion type Fab (trispecific Fabbody) was evaluated in accordance with the following protocol based on the principle shown in Fig. 20A.
(1) 50 µL of a purified second target antigen solution diluted to 10 µg/mL was added to a 96 well plate and the plate was allowed to stand at room temperature for 3 hours.
(2) After the purified second target antigen solution was removed, 200 µL/well of Blocking One (product of Nacalai Tesque) diluted to 10-fold with TBS (TBS; 20 mM Tris-HCI, 150 mM NaCl, pH 7.5) was added and the plate was allowed to stand overnight at 4°C.
(3) The plate was washed once with 200 µL/well of TBS.
(3) 50 µL of an expression supernatant of the trispecific peptide fusion type was added and the plate was allowed to stand at room temperature for 2.5 hours.
(4) The plate was washed three times with 200 µL/well of TBS.
(5) 50 µL of a supernatant expressing an AP fusion first target antigen was added and the plate was allowed to stand at room temperature for 2 hours.
(6) The plate was washed four times with 200 µL/well of TBS and lastly, 100 µL/well of TBS was added.
(7) After 100 µL/well of a chromogenic substrate (Phosphatase substrate, product of Sigma Aldrich) was added and the plate was allowed to stand at 37°C for 30 minutes, absorbance at 405 nm of the solution in each well was measured. The results are shown in Fig. 46B.

The trispecific peptide fusion type Fab was captured on the plate having each of the second target antigens immobilized thereon and was bound to the first target antigen-AP fusion protein added later to cause the substrate to develop color. On the other hand, the N1_Fab having no peptide inserted therein did not give a positive signal. This shows that Fabbody, a trispecific peptide fusion type Fab, is capable of simultaneously binding to plexin B1 and neuropilin or Met and neuropilin. In other words, the present test results suggest that the Fabbody thus produced has trispecificity.

A peptide fusion protein obtained by fusing one bioactive peptide with the Fab region, that is, antigen-binding site of IgG, can have new binding ability based on the bioactivity of the fused bioactive peptide while making use of the essential antigen-binding site of the Fab region.

When Fab is used as a protein having a Fab region which is an antigen-binding site of IgG, a bispecific Fab was constructed easily. The term "bispecific Fab" as used herein means a kind of Fabbody.

Since Fab exists as a hetero dimer, amino acid sequences derived from respectively different bioactive peptides can be inserted into the H chain and the L chain. This makes it possible to develop multispecific Fabs such as trispecific Fab or tetra- or higher specific Fab. In practice, it has been confirmed that with regard to the trispecific Fab, a peptide fusion protein can have new binding ability based on the bioactivities of two kinds of the fused bioactive peptides while making use of the original antigen binding site of the Fab region.

It is also possible to develop a multispecific antibody by inserting an amino acid sequence derived from a bioactive peptide into not only IgG or Fab but also a bispecific antibody different in antigen determination site as shown in Fig. 3 to Fig. 5 or a small fragment or derivative thereof.

When a bioactive peptide has an agonist activity, it leads to development of Fab having the agonist activity of the bioactivity-derived peptide. Even when the bioactive peptide does not have an agonist activity, an artificial protein having an agonist activity can be obtained by obtaining it as the artificial protein of the present invention having the bioactive peptide fused therein.

It has been confirmed that even in the Fv region of Fab, insertion of an amino acid sequence derived from a bioactive peptide in both the L chain and the H chain and retention of its activity can be achieved so that a bioactive peptide can be inserted also into a fragment having at least an Fv region such as scFv, Diabody, or taFv.

## Claims

1. An artificial protein having a bioactive peptide fused with a site, of a protein having at least an antigen-binding region of an antibody, present in a folded portion of a secondary structure in a structural region having an antigen-binding activity and exposed on a surface of the protein.

2. The artificial protein according to Claim 1, retaining an antigen-binding activity derived from the antibody and endowed with a function derived from the bioactive peptide.

3. The artificial protein according to Claim 1 or 2, wherein the bioactive peptide is a cyclic peptide or a linear peptide.

4. The artificial protein according to any one of Claims 1 to 3, wherein the protein having at least an antigen-binding region of an antibody is Fv, Fab, scFv, IgG, Diabody, or taFv.

5. The artificial protein according to any one of Claims 1 to 4, wherein the site present in a folded portion of a secondary structure in a structural region having an antigen-binding activity and exposed on a surface of a protein is present in a loop region or an elbow region in an antibody Fab fragment.

6. The artificial protein according to any one of Claims 1 to 5, having m ultispecificity.

7. A method of presenting a bioactive peptide on a protein having at least an antigen-binding region of an antibody, comprising fusing the bioactive peptide with a site of the protein present in a folded portion of a secondary structure in a structural region having an antigen-binding activity and exposed on a surface of the protein.

8. The method of presenting a bioactive peptide on a protein according to Claim 7, comprising allowing the protein to retain an antigen binding activity derived from the antibody and giving a function derived from the bioactive peptide to the protein.

9. The method of presenting a bioactive peptide on a protein according to Claim 7 or 8, wherein the bioactive peptide is a cyclic peptide or a linear peptide.

10. The method of presenting a bioactive peptide on a protein according to any one of Claims 7 to 9, wherein the protein having at least an antigen-binding region of an antibody is Fv, Fab, scFv, IgG, Diabody, or taFv.

11. The method of presenting a bioactive peptide on a protein according to any one of Claims 7 to 10, wherein the site present in a folded portion of a secondary structure in a structural region having an antigen-binding activity and exposed on a surface of the protein is present in a loop region or an elbow region in an antibody Fab fragment.


SEQUENCE LISTING

<110>  MiraBiologics Inc.

<120>  Artificial protein containing an antibody antigen-binding region,
        fused with bioactive peptides

<130>  M2694AKP0001

<150>  US62/669,468
<151>  2018-05-10

<160>  53

<170>  PatentIn version 3.5

<210>  1
<211>  469
<212>  PRT
<213>  Artificial Sequence

<220>
<223>  YW64.3 (human, IgG1) heavy chain

<400>  1

Met Glu Ser Gln Thr Gln Val Leu Met Phe Leu Leu Leu Trp Val Ser
1               5                   10                  15

Gly Ala Ala Ala Glu Val Gln Leu Val Glu Ser Gly Gly Gly Leu Val
            20                  25                  30

Gln Pro Gly Gly Ser Leu Arg Leu Ser Cys Ala Ala Ser Gly Phe Ser
        35                  40                  45

Phe Ser Ser Glu Pro Ile Ser Trp Val Arg Gln Ala Pro Gly Lys Gly
    50                  55                  60

Leu Glu Trp Val Ser Ser Ile Thr Gly Lys Asn Gly Tyr Thr Tyr Tyr
65                  70                  75                  80

Ala Asp Ser Val Lys Gly Arg Phe Thr Ile Ser Ala Asp Thr Ser Lys
                85                  90                  95

Asn Thr Ala Tyr Leu Gln Met Asn Ser Leu Arg Ala Glu Asp Thr Ala
            100                 105                 110

Val Tyr Tyr Cys Ala Arg Trp Gly Lys Lys Val Tyr Gly Met Asp Val
        115                 120                 125

Trp Gly Gln Gly Thr Leu Val Thr Val Ser Ser Ala Ser Thr Lys Gly
    130                 135                 140

Pro Ser Val Phe Pro Leu Ala Pro Ser Ser Lys Ser Thr Ser Gly Gly

145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val Glu Pro Lys
225                 230                 235                 240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Pro Ile Glu Lys Thr Ile Ser Lys Ala Lys Gly Gln Pro Arg Glu Pro
        355                 360                 365

Gln Val Tyr Thr Leu Pro Pro Ser Arg Asp Glu Leu Thr Lys Asn Gln
    370                 375                 380

Val Ser Leu Thr Cys Leu Val Lys Gly Phe Tyr Pro Ser Asp Ile Ala
385                 390                 395                 400

Val Glu Trp Glu Ser Asn Gly Gln Pro Glu Asn Asn Tyr Lys Thr Thr
                405                 410                 415

Pro Pro Val Leu Asp Ser Asp Gly Ser Phe Phe Leu Tyr Ser Lys Leu
            420                 425                 430

Thr Val Asp Lys Ser Arg Trp Gln Gln Gly Asn Val Phe Ser Cys Ser
        435                 440                 445

Val Met His Glu Ala Leu His Asn His Tyr Thr Gln Lys Ser Leu Ser
    450                 455                 460

Leu Ser Pro Gly Lys
465

<210> 2
<211> 255
<212> PRT
<213> Artificial Sequence

<220>
<223> YW64.3 (human, IgG1) heavy chain Fab construct

<400> 2

Met Glu Ser Gln Thr Gln Val Leu Met Phe Leu Leu Leu Trp Val Ser
1               5                   10                  15

Gly Ala Ala Ala Glu Val Gln Leu Val Glu Ser Gly Gly Gly Leu Val
            20                  25                  30

Gln Pro Gly Gly Ser Leu Arg Leu Ser Cys Ala Ala Ser Gly Phe Ser
        35                  40                  45

Phe Ser Ser Glu Pro Ile Ser Trp Val Arg Gln Ala Pro Gly Lys Gly
    50                  55                  60

Leu Glu Trp Val Ser Ser Ile Thr Gly Lys Asn Gly Tyr Thr Tyr Tyr
65                  70                  75                  80

Ala Asp Ser Val Lys Gly Arg Phe Thr Ile Ser Ala Asp Thr Ser Lys
                85                  90                  95

Asn Thr Ala Tyr Leu Gln Met Asn Ser Leu Arg Ala Glu Asp Thr Ala
            100                 105                 110

Val Tyr Tyr Cys Ala Arg Trp Gly Lys Lys Val Tyr Gly Met Asp Val
        115                 120                 125

Trp Gly Gln Gly Thr Leu Val Thr Val Ser Ser Ala Ser Thr Lys Gly

130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val Glu Pro Lys
225                 230                 235                 240

Ser Cys Asp Lys Thr His Thr Ser Arg His His His His His His
                245                 250                 255

<210>  3
<211>  234
<212>  PRT
<213>  Artificial Sequence

<220>
<223>  YW64.3 (human, kappa) light chain

<400>  3

Met Glu Ser Gln Thr Gln Val Leu Met Phe Leu Leu Leu Trp Val Ser
1               5                   10                  15

Gly Ala Ala Ala Asp Ile Gln Met Thr Gln Ser Pro Ser Ser Leu Ser
            20                  25                  30

Ala Ser Val Gly Asp Arg Val Thr Ile Thr Cys Arg Ala Ser Gln Ser
        35                  40                  45

Ile Ser Ser Tyr Leu Ala Trp Tyr Gln Gln Lys Pro Gly Lys Ala Pro
    50                  55                  60

Lys Leu Leu Ile Tyr Gly Ala Ser Ser Arg Ala Ser Gly Val Pro Ser
65                  70                  75                  80

Arg Phe Ser Gly Ser Gly Ser Gly Thr Asp Phe Thr Leu Thr Ile Ser
                85                  90                  95

Ser Leu Gln Pro Glu Asp Phe Ala Thr Tyr Tyr Cys Gln Gln Tyr Met
            100                 105                 110

Ser Val Pro Ile Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210> 4
<211> 14
<212> PRT
<213> Artificial Sequence

<220>
<223> mP6-9

<400> 4

Arg Pro Tyr Ile Glu Arg Trp Thr Gly Arg Leu Ile Val Cys
1               5                   10

<210> 5
<211> 14
<212> PRT
<213> Artificial Sequence

<220>
<223> inserted peptide (mP6-8)

<400> 5

Trp Arg Pro Tyr Ile Glu Arg Trp Thr Gly Arg Leu Ile Val

1               5                   10


<210>  6
<211>  26
<212>  PRT
<213>  Artificial Sequence

<220>
<223>  partial amino acid sequence (mP6-8 insertion)

<400>  6

Val Glu Ile Lys Arg Gly Trp Arg Pro Tyr Ile Glu Arg Trp Thr Gly
1               5                   10                  15


Arg Leu Ile Val Gly Thr Val Ala Ala Pro
            20                  25


<210>  7
<211>  27
<212>  PRT
<213>  Artificial Sequence

<220>
<223>  partial amino acid sequence (mP6-8 insertion)

<400>  7

Glu Gln Leu Lys Ser Gly Trp Arg Pro Tyr Ile Glu Arg Trp Thr Gly
1               5                   10                  15


Arg Leu Ile Val Gly Gly Gly Thr Ala Ser Val
            20                  25


<210>  8
<211>  27
<212>  PRT
<213>  Artificial Sequence

<220>
<223>  partial amino acid sequence (mP6-8 insertion)

<400>  8

Gln Trp Lys Val Asp Gly Trp Arg Pro Tyr Ile Glu Arg Trp Thr Gly
1               5                   10                  15


Arg Leu Ile Val Gly Gly Asn Ala Leu Gln Ser
            20                  25


<210>  9
<211>  27
<212>  PRT
<213>  Artificial Sequence

<220>

<223>  partial amino acid sequence (mP6-8 insertion)

<400>  9

Asn Ala Leu Gln Ser Gly Trp Arg Pro Tyr Ile Glu Arg Trp Thr Gly
1               5                   10                  15

Arg Leu Ile Val Gly Gly Gly Asn Ser Gln Glu
            20                  25

<210>  10
<211>  27
<212>  PRT
<213>  Artificial Sequence

<220>
<223>  partial amino acid sequence (mP6-8 insertion)

<400>  10

Ala Asp Tyr Glu Lys Gly Trp Arg Pro Tyr Ile Glu Arg Trp Thr Gly
1               5                   10                  15

Arg Leu Ile Val Gly Gly His Lys Val Tyr Ala
            20                  25

<210>  11
<211>  27
<212>  PRT
<213>  Artificial Sequence

<220>
<223>  partial amino acid sequence (mP6-8 insertion)

<400>  11

Leu Ser Ala Ser Val Gly Trp Arg Pro Tyr Ile Glu Arg Trp Thr Gly
1               5                   10                  15

Arg Leu Ile Val Gly Gly Gly Asp Arg Val Thr
            20                  25

<210>  12
<211>  27
<212>  PRT
<213>  Artificial Sequence

<220>
<223>  partial amino acid sequence (mP6-8 insertion)

<400>  12

Ser Ser Arg Ala Ser Gly Trp Arg Pro Tyr Ile Glu Arg Trp Thr Gly
1               5                   10                  15

Arg Leu Ile Val Gly Gly Gly Val Pro Ser Arg

20                  25


<210>  13
<211>  27
<212>  PRT
<213>  Artificial Sequence

<220>
<223>  partial amino acid sequence (mP6-8 insertion)

<400>  13

Ser Gly Val Pro Ser Gly Trp Arg Pro Tyr Ile Glu Arg Trp Thr Gly
1               5                   10                  15


Arg Leu Ile Val Gly Gly Arg Phe Ser Gly Ser
            20                  25


<210>  14
<211>  27
<212>  PRT
<213>  Artificial Sequence

<220>
<223>  partial amino acid sequence (mP6-8 insertion)

<400>  14

Thr Leu Thr Ile Ser Gly Trp Arg Pro Tyr Ile Glu Arg Trp Thr Gly
1               5                   10                  15


Arg Leu Ile Val Gly Gly Ser Leu Gln Pro Glu
            20                  25


<210>  15
<211>  27
<212>  PRT
<213>  Artificial Sequence

<220>
<223>  partial amino acid sequence (mP6-8 insertion)

<400>  15

Ser Ser Leu Gln Pro Gly Trp Arg Pro Tyr Ile Glu Arg Trp Thr Gly
1               5                   10                  15


Arg Leu Ile Val Gly Gly Glu Asp Phe Ala Thr
            20                  25


<210>  16
<211>  27
<212>  PRT
<213>  Artificial Sequence

<220>

<223>  partial amino acid sequence (mP6-8 insertion)

<400>  16

Glu Gln Asp Ser Lys Gly Trp Arg Pro Tyr Ile Glu Arg Trp Thr Gly
1               5                   10                  15

Arg Leu Ile Val Gly Gly Asp Ser Thr Tyr Ser
            20                  25

<210>  17
<211>  27
<212>  PRT
<213>  Artificial Sequence

<220>
<223>  partial amino acid sequence (mP6-8 insertion)

<400>  17

His Gln Gly Leu Ser Gly Trp Arg Pro Tyr Ile Glu Arg Trp Thr Gly
1               5                   10                  15

Arg Leu Ile Val Gly Gly Ser Pro Val Thr Lys
            20                  25

<210>  18
<211>  27
<212>  PRT
<213>  Artificial Sequence

<220>
<223>  partial amino acid sequence (mP6-8 insertion)

<400>  18

Glu Val Thr His Gln Gly Trp Arg Pro Tyr Ile Glu Arg Trp Thr Gly
1               5                   10                  15

Arg Leu Ile Val Gly Gly Gly Leu Ser Ser Pro
            20                  25

<210>  19
<211>  27
<212>  PRT
<213>  Artificial Sequence

<220>
<223>  partial amino acid sequence (mP6-8 insertion)

<400>  19

Tyr Gln Gln Lys Pro Gly Trp Arg Pro Tyr Ile Glu Arg Trp Thr Gly
1               5                   10                  15

Arg Leu Ile Val Gly Gly Gly Lys Ala Pro Lys

20                  25


<210>  20
<211>  26
<212>  PRT
<213>  Artificial Sequence

<220>
<223>  partial amino acid sequence (mP6-8 insertion)

<400>  20

Val Thr Val Ser Ser Gly Trp Arg Pro Tyr Ile Glu Arg Trp Thr Gly
1               5                   10                  15


Arg Leu Ile Val Gly Ala Ser Thr Lys Gly
            20                  25


<210>  21
<211>  27
<212>  PRT
<213>  Artificial Sequence

<220>
<223>  partial amino acid sequence (mP6-8 insertion)

<400>  21

Ser Lys Ser Thr Ser Gly Trp Arg Pro Tyr Ile Glu Arg Trp Thr Gly
1               5                   10                  15


Arg Leu Ile Val Gly Gly Gly Gly Thr Ala Ala
            20                  25


<210>  22
<211>  27
<212>  PRT
<213>  Artificial Sequence

<220>
<223>  partial amino acid sequence (mP6-8 insertion)

<400>  22

Val Ser Trp Asn Ser Gly Trp Arg Pro Tyr Ile Glu Arg Trp Thr Gly
1               5                   10                  15


Arg Leu Ile Val Gly Gly Gly Ala Leu Thr Ser
            20                  25


<210>  23
<211>  27
<212>  PRT
<213>  Artificial Sequence

<220>

<223>  partial amino acid sequence (mP6-8 insertion)

<400>  23

Ser Gly Ala Leu Thr Gly Trp Arg Pro Tyr Ile Glu Arg Trp Thr Gly
1               5                   10                  15

Arg Leu Ile Val Gly Gly Ser Gly Val His Thr
            20                  25

<210>  24
<211>  27
<212>  PRT
<213>  Artificial Sequence

<220>
<223>  partial amino acid sequence (mP6-8 insertion)

<400>  24

Ser Ser Ser Leu Gly Gly Trp Arg Pro Tyr Ile Glu Arg Trp Thr Gly
1               5                   10                  15

Arg Leu Ile Val Gly Gly Thr Gln Thr Tyr Ile
            20                  25

<210>  25
<211>  27
<212>  PRT
<213>  Artificial Sequence

<220>
<223>  partial amino acid sequence (mP6-8 insertion)

<400>  25

Gly Leu Val Gln Pro Gly Trp Arg Pro Tyr Ile Glu Arg Trp Thr Gly
1               5                   10                  15

Arg Leu Ile Val Gly Gly Gly Gly Ser Leu Arg
            20                  25

<210>  26
<211>  27
<212>  PRT
<213>  Artificial Sequence

<220>
<223>  partial amino acid sequence (mP6-8 insertion)

<400>  26

Thr Tyr Tyr Ala Asp Gly Trp Arg Pro Tyr Ile Glu Arg Trp Thr Gly
1               5                   10                  15

Arg Leu Ile Val Gly Gly Ser Val Lys Gly Arg

20                  25


<210>  27
<211>  27
<212>  PRT
<213>  Artificial Sequence

<220>
<223>  partial amino acid sequence (mP6-8 insertion)

<400>  27

Asp Ser Val Lys Gly Gly Trp Arg Pro Tyr Ile Glu Arg Trp Thr Gly
1               5                   10                  15


Arg Leu Ile Val Gly Gly Arg Phe Thr Ile Ser
            20                  25


<210>  28
<211>  29
<212>  PRT
<213>  Artificial Sequence

<220>
<223>  partial amino acid sequence (mP6-8 insertion)

<400>  28

Tyr Leu Gln Met Asn Ala Gly Trp Arg Pro Tyr Ile Glu Arg Trp Thr
1               5                   10                  15


Gly Arg Leu Ile Val Gly Gly Ser Asx Leu Arg Ala Glu
            20                  25


<210>  29
<211>  27
<212>  PRT
<213>  Artificial Sequence

<220>
<223>  partial amino acid sequence (mP6-8 insertion)

<400>  29

Met Asn Ser Leu Arg Gly Trp Arg Pro Tyr Ile Glu Arg Trp Thr Gly
1               5                   10                  15


Arg Leu Ile Val Gly Gly Ala Glu Asp Thr Ala
            20                  25


<210>  30
<211>  27
<212>  PRT
<213>  Artificial Sequence

<220>

<223>  partial amino acid sequence (mP6-8 insertion)

<400>  30

Ala Val Leu Gln Ser Gly Trp Arg Pro Tyr Ile Glu Arg Trp Thr Gly
1               5                   10                  15

Arg Leu Ile Val Gly Gly Ser Gly Leu Tyr Ser
            20                  25

<210>  31
<211>  27
<212>  PRT
<213>  Artificial Sequence

<220>
<223>  partial amino acid sequence (mP6-8 insertion)

<400>  31

Asn His Lys Pro Ser Gly Trp Arg Pro Tyr Ile Glu Arg Trp Thr Gly
1               5                   10                  15

Arg Leu Ile Val Gly Gly Asn Thr Lys Val Asp
            20                  25

<210>  32
<211>  27
<212>  PRT
<213>  Artificial Sequence

<220>
<223>  partial amino acid sequence (mP6-8 insertion)

<400>  32

Val Arg Gln Ala Pro Gly Trp Arg Pro Tyr Ile Glu Arg Trp Thr Gly
1               5                   10                  15

Arg Leu Ile Val Gly Gly Gly Lys Gly Leu Glu
            20                  25

<210>  33
<211>  8
<212>  PRT
<213>  Artificial Sequence

<220>
<223>  FLAG tag

<400>  33

Asp Tyr Lys Asp Asp Asp Asp Lys
1               5

<210>  34

<211>  15
<212>  PRT
<213>  Artificial Sequence

<220>
<223>  aMD4

<400>  34

Arg Gln Phe Asn Arg Arg Thr His Glu Val Trp Asn Leu Asp Cys
1               5                   10                  15


<210>  35
<211>  15
<212>  PRT
<213>  Artificial Sequence

<220>
<223>  inserted peptide (aMD4)

<400>  35

Tyr Arg Gln Phe Asn Arg Arg Thr His Glu Val Trp Asn Leu Asp
1               5                   10                  15


<210>  36
<211>  29
<212>  PRT
<213>  Artificial Sequence

<220>
<223>  partial amino acid sequence (aMD4 insertion)

<400>  36

Val Glu Ile Lys Arg Gly Gly Tyr Arg Gln Phe Asn Arg Arg Thr His
1               5                   10                  15


Glu Val Trp Asn Leu Asp Gly Gly Thr Val Ala Ala Pro
            20                  25


<210>  37
<211>  29
<212>  PRT
<213>  Artificial Sequence

<220>
<223>  partial amino acid sequence (aMD4 insertion)

<400>  37

Asn Ala Leu Gln Ser Gly Gly Tyr Arg Gln Phe Asn Arg Arg Thr His
1               5                   10                  15


Glu Val Trp Asn Leu Asp Gly Gly Gly Asn Ser Gln Glu
            20                  25

<210>  38
<211>  29
<212>  PRT
<213>  Artificial Sequence

<220>
<223>  partial amino acid sequence (aMD4 insertion)

<400>  38

Leu Ser Ala Ser Val Gly Gly Tyr Arg Gln Phe Asn Arg Arg Thr His
1               5                   10                  15

Glu Val Trp Asn Leu Asp Gly Gly Gly Asp Arg Val Thr
            20                  25

<210>  39
<211>  29
<212>  PRT
<213>  Artificial Sequence

<220>
<223>  partial amino acid sequence (aMD4 insertion)

<400>  39

Ser Ser Arg Ala Ser Gly Gly Tyr Arg Gln Phe Asn Arg Arg Thr His
1               5                   10                  15

Glu Val Trp Asn Leu Asp Gly Gly Gly Val Pro Ser Arg
            20                  25

<210>  40
<211>  29
<212>  PRT
<213>  Artificial Sequence

<220>
<223>  partial amino acid sequence (aMD4 insertion)

<400>  40

Ser Gly Val Pro Ser Gly Gly Tyr Arg Gln Phe Asn Arg Arg Thr His
1               5                   10                  15

Glu Val Trp Asn Leu Asp Gly Gly Arg Phe Ser Gly Ser
            20                  25

<210>  41
<211>  29
<212>  PRT
<213>  Artificial Sequence

<220>
<223>  partial amino acid sequence (aMD4 insertion)

<400>  41

Ser Ser Leu Gln Pro Gly Gly Tyr Arg Gln Phe Asn Arg Arg Thr His
1               5                   10                  15

Glu Val Trp Asn Leu Asp Gly Gly Glu Asp Phe Ala Thr
            20                  25

<210>  42
<211>  29
<212>  PRT
<213>  Artificial Sequence

<220>
<223>  partial amino acid sequence (aMD4 insertion)

<400>  42

Glu Gln Asp Ser Lys Gly Gly Tyr Arg Gln Phe Asn Arg Arg Thr His
1               5                   10                  15

Glu Val Trp Asn Leu Asp Gly Gly Asp Ser Thr Tyr Ser
            20                  25

<210>  43
<211>  29
<212>  PRT
<213>  Artificial Sequence

<220>
<223>  partial amino acid sequence (aMD4 insertion)

<400>  43

Tyr Gln Gln Lys Pro Gly Gly Tyr Arg Gln Phe Asn Arg Arg Thr His
1               5                   10                  15

Glu Val Trp Asn Leu Asp Gly Gly Gly Lys Ala Pro Lys
            20                  25

<210>  44
<211>  29
<212>  PRT
<213>  Artificial Sequence

<220>
<223>  partial amino acid sequence (aMD4 insertion)

<400>  44

Val Thr Val Ser Ser Gly Gly Tyr Arg Gln Phe Asn Arg Arg Thr His
1               5                   10                  15

Glu Val Trp Asn Leu Asp Gly Gly Ala Ser Thr Lys Gly
            20                  25

<210>  45
<211>  29
<212>  PRT
<213>  Artificial Sequence

<220>
<223>  partial amino acid sequence (aMD4 insertion)

<400>  45

Ser Lys Ser Thr Ser Gly Gly Tyr Arg Gln Phe Asn Arg Arg Thr His
1               5                   10                  15

Glu Val Trp Asn Leu Asp Gly Gly Gly Gly Thr Ala Ala
            20                  25

<210>  46
<211>  29
<212>  PRT
<213>  Artificial Sequence

<220>
<223>  partial amino acid sequence (aMD4 insertion)

<400>  46

Val Ser Trp Asn Ser Gly Gly Tyr Arg Gln Phe Asn Arg Arg Thr His
1               5                   10                  15

Glu Val Trp Asn Leu Asp Gly Gly Gly Ala Leu Thr Ser
            20                  25

<210>  47
<211>  29
<212>  PRT
<213>  Artificial Sequence

<220>
<223>  partial amino acid sequence (aMD4 insertion)

<400>  47

Ser Ser Ser Leu Gly Gly Gly Tyr Arg Gln Phe Asn Arg Arg Thr His
1               5                   10                  15

Glu Val Trp Asn Leu Asp Gly Gly Thr Gln Thr Tyr Ile
            20                  25

<210>  48
<211>  29
<212>  PRT
<213>  Artificial Sequence

<220>
<223>  partial amino acid sequence (aMD4 insertion)

<400>  48

Thr Tyr Tyr Ala Asp Gly Gly Tyr Arg Gln Phe Asn Arg Arg Thr His
1               5                   10                  15

Glu Val Trp Asn Leu Asp Gly Gly Ser Val Lys Gly Arg
            20                  25

<210>  49
<211>  29
<212>  PRT
<213>  Artificial Sequence

<220>
<223>  partial amino acid sequence (aMD4 insertion)

<400>  49

Ala Val Leu Gln Ser Gly Gly Tyr Arg Gln Phe Asn Arg Arg Thr His
1               5                   10                  15

Glu Val Trp Asn Leu Asp Gly Gly Ser Gly Leu Tyr Ser
            20                  25

<210>  50
<211>  29
<212>  PRT
<213>  Artificial Sequence

<220>
<223>  partial amino acid sequence (aMD4 insertion)

<400>  50

Asn His Lys Pro Ser Gly Gly Tyr Arg Gln Phe Asn Arg Arg Thr His
1               5                   10                  15

Glu Val Trp Asn Leu Asp Gly Gly Asn Thr Lys Val Asp
            20                  25

<210>  51
<211>  29
<212>  PRT
<213>  Artificial Sequence

<220>
<223>  partial amino acid sequence (aMD4 insertion)

<400>  51

Val Arg Gln Ala Pro Gly Gly Tyr Arg Gln Phe Asn Arg Arg Thr His
1               5                   10                  15

Glu Val Trp Asn Leu Asp Gly Gly Gly Lys Gly Leu Glu
            20                  25

<210>  52
<211>  14
<212>  PRT
<213>  Artificial Sequence

<220>
<223>  aMD5

<400>  52

Trp Tyr Tyr Ala Trp Asp Gln Thr Tyr Lys Ala Phe Pro Cys
1               5                   10


<210>  53
<211>  14
<212>  PRT
<213>  Artificial Sequence

<220>
<223>  inserted peptide (aMD5)

<400>  53

Tyr Trp Tyr Tyr Ala Trp Asp Gln Thr Tyr Lys Ala Phe Pro
1               5                   10